# EUROPEAN PATENT SPECIFICATION

(11) **EP 4 126 052 B1**
(45) Date of publication and mention of the grant of the patent: **24.12.2025**
(21) Application number: 21719056.0
(22) Date of filing: 29.03.2021
(51) Int. Cl.: A61K 45/06, A61K 47/64, A61P 35/00

(54) **USE OF CONJUGATES COMPRISING TUMOUR-SELECTIVE LIGANDS AND GROUPS CAPABLE OF RELEASING CARBON MONOXIDE (CO), FOR EXERTING IMMUNOMODULATORY EFFECTS IN CANCER TREATMENT**
VERWENDUNG VON KONJUGATEN MIT TUMORSELEKTIVEN LIGANDEN UND GRUPPEN ZUR FREISETZUNG VON KOHLENMONOXID (CO) ZUR AUSÜBUNG IMMUNMODULATORISCHER EFFEKTE BEI DER KREBSBEHANDLUNG
UTILISATION DE CONJUGUÉS COMPRENANT DES LIGANDS SÉLECTIFS DES TUMEURS ET DES GROUPES POUVANT LIBÉRER DU MONOXYDE DE CARBONE (CO), POUR EXERCER DES EFFETS IMMUNOMODULATEURS DANS LE TRAITEMENT DU CANCER

(30) Priority: 27.03.2020 GB 202004514
(43) Date of publication of application: 08.02.2023
(73) Proprietor: Fundação GIMM - Gulbenkian Institute for Molecular Medicine, 1649-035 Alvalade, Lisboa (PT); Faculdade de Medicina da Universidade de Lisboa, 1649-028 Lisboa (PT)
(72) Inventor: LABÃO ALPIARÇA SOUSA DE ALMEIDA, Carlos Diogo, 1649-028 Lisboa (PT); LOPES BERNARDES, Gonçalo José, 1649-028 Lisboa (PT)
(74) Representative: Grund, Martin
(86) International application number: PCT/EP2021/058146
(87) International publication number: WO 2021/191464

(56) References cited:
- WO-A1-2017/007955
- CN-A- 108 567 980
- US-A1- 2021 060 167
- KAWAHARA BRIAN ET AL: "Diminished viability of human ovarian cancer cells by antigen-specific delivery of carbon monoxide with a family of photoactivatable antibody-photoCORM conjugates", CHEMICAL SCIENCE, vol. 11, no. 2, 2 January 2020 (2020-01-02), United Kingdom, pages 467 - 473, XP055815534, ISSN: 2041-6520, Retrieved from the Internet <URL:https://pubs.rsc.org/en/content/articlepdf/2020/sc/c9sc03166a> DOI: 10.1039/C9SC03166A
- ODACHOWSKI MATYLDA ET AL: "A review on 1,1-bis(diphenylphosphino)methane bridged homo- and heterobimetallic complexes for anticancer applications: Synthesis, structure, and cytotoxicity", EUROPEAN JOURNAL OF MEDICINAL CHEMISTRY, ELSEVIER, AMSTERDAM, NL, vol. 204, 15 July 2020 (2020-07-15), XP086262485, ISSN: 0223-5234, [retrieved on 20200715], DOI: 10.1016/J.EJMECH.2020.112613
- OPOKU-DAMOAH YAW ET AL: "Lipid-encapsulated upconversion nanoparticle for near-infrared light-mediated carbon monoxide release for cancer gas therapy", EUROPEAN JOURNAL OF PHARMACEUTICS AND BIOPHARMACEUTICS, ELSEVIER SCIENCE PUBLISHERS B.V., AMSTERDAM, NL, vol. 158, 1 December 2020 (2020-12-01), pages 211 - 221, XP086463985, ISSN: 0939-6411, [retrieved on 20201201], DOI: 10.1016/J.EJPB.2020.11.014

## Description

### Field

This invention relates to a conjugate for use in a method of reducing tumour immunosuppression in immunosuppressive cancers.

### Background

Cancer is one of the leading causes of death worldwide and incidence rates have systematically increased over time [1]. Advances in cancer research have extended our understanding of the role played by the immune system on host protection against tumour development. Not only is the immune system able to recognise a plethora of antigens derived from foreign organisms or components, but it also targets tumour cells [2]. However, cancer cells possess many mechanisms of immune evasion by which they subvert and escape immune cell-mediated recognition and destruction. Targeting the mechanisms of tumour immunity has led to the development of novel therapeutic approaches that are yielding promising results in clinical trials. Immunotherapy is a promising approach in cancer treatment that harnesses the immune system to specifically target tumour cells [2-4]. However, immunotherapeutics are not effective in all cancer patients, and often do not yield complete or durable clinical responses [5, 6]. Therefore, new tactics in promoting anti-tumour immune responses are desired.

CN 108 567 980 A discloses an anti-tumour effect of carbon monoxide releasing molecule CORM@G3DSP-Ce6 in a breast cancer 4T1 model. The mechanism of action was observed to be a reduction of microvessel density as demonstrated by immunohistochemical staining of CD31.

Carbon monoxide (CO) is a gaseous mediator with several pleiotropic effects that has been observed to have beneficial properties in some cancer models [7-10]. However, these observations have not been consistently observed, with CO also being associated with tumour growth or progression in some models [65]. Further, the toxic nature of CO means that it must be delivered to target tissues in a precise and controlled way, with biologically appropriate doses. Nonetheless, as the vast majority of these studies have focused on its direct effects in cancer cells, the role of CO in cancer immunity is virtually unknown.

The exogenous delivery of CO has to be tightly controlled and given at very low doses, as exposure to high concentrations of CO is severely poisonous. CO-releasing molecules or CORMs have been developed for systemic administration of higher therapeutic doses. CORMs have been tested in different disease models [7-10]. The molecular mechanisms behind the effect of CO on the immune system and in tumour growth are not fully understood. CORMs have no tissue or cell selectivity and may potentially release CO in circulation, and conjugation with a protein or antibody carrier may enable a more controlled delivery at specific tissues. For example, a photoCORM was previously conjugated with commercially available mouse antibodies [11]. However, their potential anti-cancer effects were only studied in cancer cells directly and not in co-culture with immune cells or *in vivo.*

There is a need for new, improved, and specific treatments for immunosuppressive cancers, as well as a need for compositions that provide for the effective delivery of CO for the treatment of tumours.

### Summary

The present inventors have unexpectedly found that CO releasing molecules, such as conjugates comprising tumour-selective ligands and groups capable of releasing carbon monoxide (CO), exert immunomodulatory effects, such as reductions in the expression of immune checkpoint molecules and the level of inhibitory macrophages. This can reduce immunosuppression in tumour tissue and can be useful, for example, in the treatment of immunosuppressive cancers or the treatment of cancers in combination with cancer immunotherapy.

The present invention is defined by the appended claims.

### Brief Description of the Figures

Figures 1a and 1b show a schematic representation of the conjugation strategy (a), and (b) the mass spectra of native rHSA (Recombumin) and rHSA-CORM (Recombumin-Ru(CO)2).
Figures 2a and 2b shows CO release in bone-marrow-derived macrophage cells (BMDMs) and colorectal carcinoma cells (CT26). (a) Microscopic images and Mean Fluorescent Intensity (MFI) of COP-1 0, 30 and 60 minutes after COP-1 addition in BMDM cells. (b) Same as (a), but in CT26 cells. Cells were previously cultured in 30 min with either 1µM of rHSA-CORM or rHSA-DMSO. Vertical dashed lines represent the timing of COP-1 addition to the cells.
Figure 3 shows CO biodistribution in mice and carboxyhaemoglobin (CO-Hb) levels in circulation 30 minutes after an i.v. injection of rHSA-CORM.

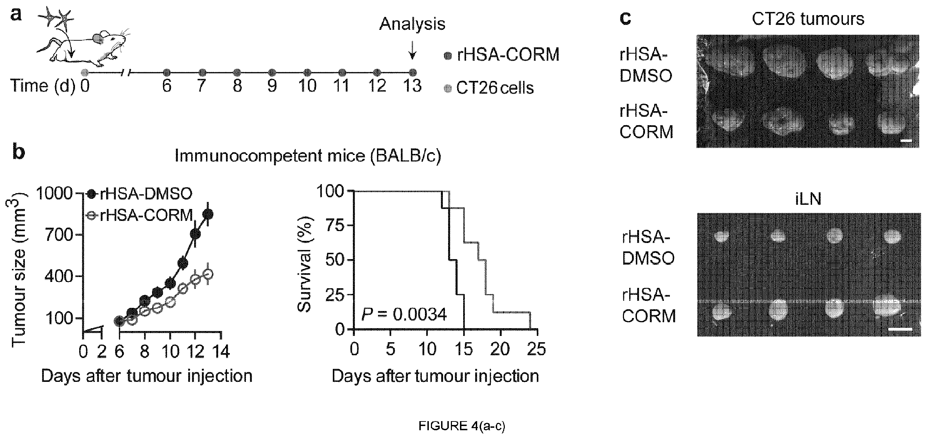

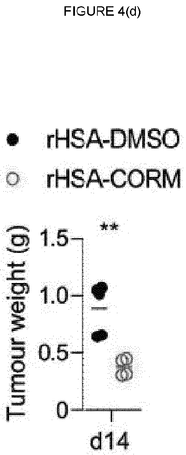

Figures 4 (a-d) show that CO prolongs survival and attenuates tumour growth in CT26 and MC38 tumours. (a) Schematic representation of the experiment. (b) Tumour growth and survival curves of CT26 (colon carcinoma) tumours after rHSA-CORM treatments (rHSA-CORM, n=8) or control treatments (rHSA-DMSO, n=8). (c) Tumours and inguinal lymph nodes (iLN) at day 13. (d) Tumour weight in colon carcinoma model with MC38 cells after treatment with rHSA-CORM or control. Statistical analysis was performed using Prism 8. For statistical test a P value of less than 0.05 considered significant unless otherwise noted. Tumour growth results were analysed by one-way ANOVA, if significant (95% confidence interval). Comparisons of the survival of tumour-bearing mice were performed using the log-rank Mantel-Cox test (95% confidence interval).
Figure 5 shows that CO does not affect tumour growth nor survival in immunocompromised mice (without a proper and functioning immune system). CT26 tumour volume and overall survival of NSG mice after daily rHSA-CORM treatments are shown (rHSA-CORM, n=6; rHSA-DMSO, n=6).
Figure 6 (a,b) shows that CO-treated mice exhibit a reduction in the expression of immune-checkpoint markers CTLA-4 and PD-1 in tumour infiltrating T cells (a), and an increase in the abundance of cells expressing TNF-α and IFNγ (b). CORM, n=4; rHSA-DMSO, n=4).

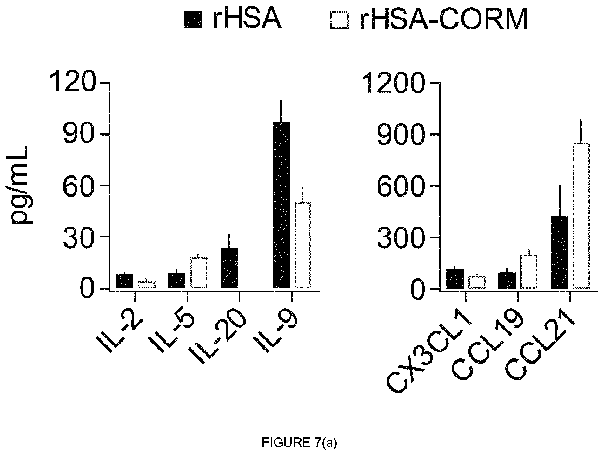

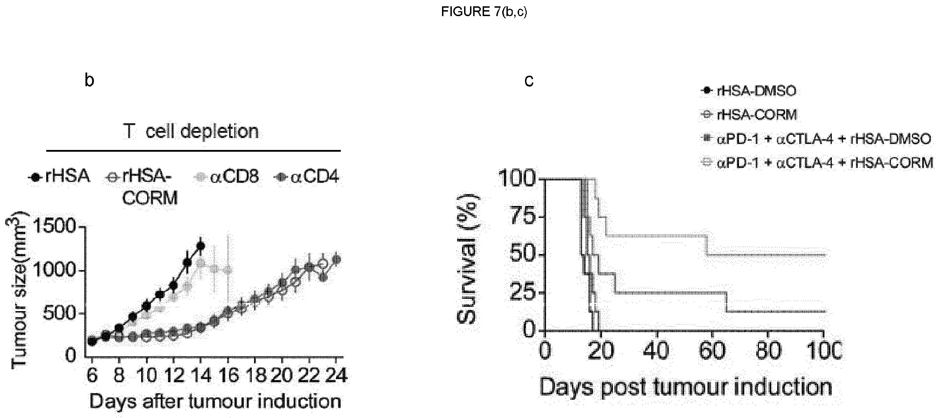

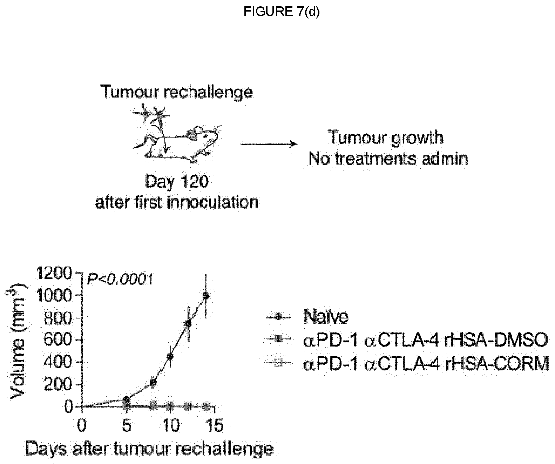

Figure 7a shows that CO affects the production of different cytokines and chemokines in tumours. The production of IL-20 appears to be completely abrogated with CO treatment. Protein quantification of cytokines (left) and chemokines (right) in tumours after rHSA-CORM treatments (rHSA-CORM, n=3; rHSA-DMSO, n=3). Figure 7b shows tumour size after CD4 and CD8 depletion in tumour-bearing BALB/c mice. Figure 7c shows survival with combination therapy between αPD-1, αCTLA-4 and rHSA-CORM (n=8). Figure 7d is a schematic representation and tumour growth curves of the CT26 rechallenge (Naïve, n=8; ICB, n=1; COMBO, n=4) in surviving mice from c.
Figure 8 shows that CO treatments reduce the abundance of monocytes and TAMs in tumours (rHSA-CORM, n=4; rHSA-DMSO, n=4).
Figure 9(a-e) shows that depletion of macrophages mimics the effects of CO treatments in CT26 tumours. (rHSA-CORM, n=4; rHSA-DMSO, n=4; rHSA-CORM+Clod Lip, n=4; rHSA-DMSO+Clod Lip, n=4). A schematic (9a), tumour volume (9b), flow cytometry plot showing macrophage depletion (9c), and DC and TAM levels (9d) are presented, (9e) shows Ki-67 expression in macrophages and production of IL-20.

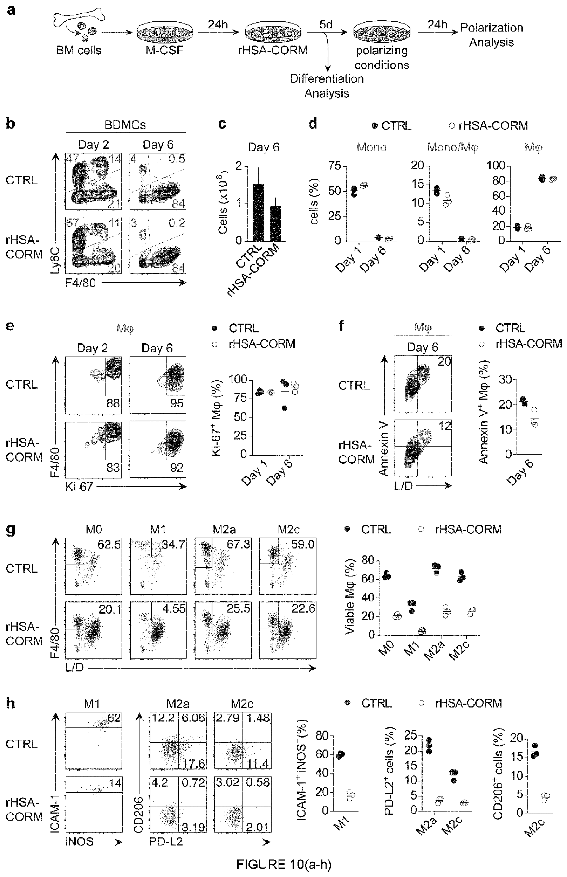

Figure 10 (a-h) shows that rHSA-CORM treatments impact bone marrow-derived macrophage viability and polarisation. (a) Schematic representation of the experiment. (b) FACS plots of BMDM at day 2 and 6 of monocyte-to-macrophage differentiation (rHSA-CORM, n=3; rHSA-DMSO, n=3). (c) Absolute number of cells after macrophage differentiation (Day 6). (d) Relative abundances of monocytes (Mono), transitioning monocytes (Mono/Mφ) and macrophages (Mφ) obtained from (b). (e) Ki-67 staining of macrophages at day 2 and 6 of differentiation. (f) Annexin V and viability staining in macrophages at day 2 and 6 of differentiation. (g) Cell viability of BMDM after polarization in rHSA-CORM or rHSA-DMSO (CTRL) conditions. (h) FACS plots of each polarising condition with their respective identification markers.
Figure 11 (a,b) shows that cell viability is affected during monocyte to macrophage transition and not after macrophage differentiation as cells only die in the presence of the conjugate during the transition and not after transition.
Figure 12(a-c) shows toxicology results for rHSA-CORM and rHSA-DMSO treated mice for various tissues. Figure 12d shows that rHSA-CORM did not stimulate metastasis.

### Detailed Description

This invention is based in part on the finding that CO releasing-conjugates reduce immunosuppression in tumours by downregulating CTLA-4 and PD-1 expression and reducing levels of tumour associated macrophages (TAMs). Tumour selective CORMs (such as CORM conjugates) may therefore be useful in the treatment of cancers, including immunosuppressive cancers, optionally in conjunction with other cancer immunotherapy.

A CO releasing-conjugate as described herein comprises a carbon monoxide releasing group conjugated to a tumour-selective ligand.

A carbon monoxide releasing group is a chemical moiety that is capable of releasing carbon monoxide (CO), following administration in vivo.

In some embodiments, a carbon monoxide releasing group may comprise one or more CO groups that are coordinated or chemically linked, for example by a covalent or other bond, to another atom, such as a metal atom. Following administration, the linkage of the CO groups to the other atom is disrupted and CO groups are released. For example, CO may be spontaneously released when the conjugate comprising the carbon monoxide releasing group is placed in aqueous solution. Release of CO by the conjugate increases the level of CO at the site of a tumour in an individual and exerts an immunomodulatory effect, as described herein.

Suitable CO releasing groups include metal carbonyl complexes in which one or more CO moieties are coordinated to a metal atom. Suitable metal carbonyl complexes include mono- di- tri- tetra- or pentacarbonyl metal complexes. For example a complex of the formula M(CO)ₙ₁, where M is a metal atom and n1 is 1, 2, 3, 4 or 5, preferably 2 or 3.

Suitable metals may include iron, molybdenum, cobalt, rhenium and ruthenium.

In some embodiments, the metal is ruthenium. For example, the CO releasing group may be a ruthenium dicarbonyl group of the formula Ru^{II}(CO)₂.

Exemplary CO releasing groups and molecules are disclosed in US Patent 7,964,220, US Patent 9,023,402, US Patent 9,062,089, and US Patent 9,163,044.

In other preferred embodiments, the CO releasing group comprises a molybdenum metal atom, as described in US 9,163,044, for example. In such embodiments, the CO releasing group comprises three CO moieties releasable from a Mo metal atom.

In the CO releasing conjugates described herein, a tumour selective ligand is conjugated, directly or indirectly, to a CO releasing group or particle comprising the CO releasing group. A tumour selective ligand is a molecule which selectively accumulates, or causes the CO releasing group(s) to accumulate, in tumours when administered in vivo i.e. the concentration of tumour selective ligand following administration to an individual with cancer is greater in tumour tissue relative to non-tumour tissue of the individual.

In some embodiments, the conjugate has the formula Ligand-[Metal(CO)n1]n₂ where n₁ is between 1 and 5 and n₂ is between 1 and 30.

The tumour selective ligand allows the CO releasing conjugate to selectively target or accumulate in tumour tissue in an individual. The tumour selective ligand is stable in circulation. Furthermore, the tumour selective ligand of the conjugate accumulates selectively in tumour tissue i.e. it shows increased concentrations in tumour tissue relative to non-tumour tissue. Accumulation of the tumour selective ligand in tumour tissue allows the CORM to act selectively in tumour tissue relative to non- tumour tissue (i.e. non-cancerous tissue in which the tumour selective ligand does not accumulate), for example to release CO in the tumour tissue and exert an immunomodulatory effect, for example an anti-immunosuppressive effect. The CO release is thus targeted to tumour tissue and does not significantly change the basal levels of CO-Hb.

The tumour-selective ligand targets the CO releasing group to tumours. A CO releasing group that is conjugated to a tumour-selective ligand may display increased accumulation in tumour tissue of the individual relative to non-conjugated CO releasing molecules i.e. the concentration in tumour tissue of a CO releasing group that is conjugated to a tumour-selective ligand may be increased relative to an unconjugated CO releasing molecule. This allows the selective release of CO by the CO releasing conjugate in tumour tissue, such that the released CO accumulates in the tumour tissue.

Suitable tumour-selective ligands include nanoparticles, peptides, proteins, polymers (biopolymers such as polysaccharides or synthetic polymers such as polyethylene glycol), aptamers, or small molecules.

In some preferred embodiments, the tumour-selective ligand is preferably a plasma protein, preferably a human plasma protein. Plasma proteins are proteins found in blood serum. Suitable plasma proteins may include antibodies, albumin, globulins, such as gamma globulin and alpha-2 macroglobulin, fibrinogen, lipoproteins, transferrin, regulatory factors, such as hormones, and clotting factors, such as prothrombin. Other tumour-selective ligands include amino acid sequences that provide a large hydrodynamic radius to increase circulatory half-time of the complex and avoid kidney filtration, such as elastin-like peptide (ELP) (see US Patent 8,334,257) and unstructured polymers such as those described in US 7,855,279.

Preferably, the tumour-selective ligand may be serum albumin. Serum albumin (ALB; Gene ID: 213) is the most abundant protein in human blood. Serum albumin regulates the colloid osmotic pressure of blood plasma and acts as a carrier protein for a wide range of endogenous molecules in the serum. Serum albumin accumulates in tumour tissue due to extravasation and targeting of neonatal Fc receptor (FCRN) and/or due to the enhanced permeability and retention (EPR) effect. It has previously been shown that 20% of the injected dose of a radio-labelled albumin derivative accumulated in rat subcutaneous tumours after 24 hours [64]. Thus, in some embodiments, the tumour-selective ligand is human serum albumin. Human serum albumin may have the reference amino acid sequence of NCBI database entry NP_000468.1 or SEQ ID NO: 1 or may be a variant of either one of these and may be encoded by the reference nucleotide sequence of NM_000477.7 or a variant thereof. Human serum albumin may have tropism towards the tumour microenvironment and may increase the serum half-life of drugs [12].

In some embodiments, the albumin amino acid sequence is at least about 75%, or at least about 80%, or at least about 85%, at least 90%, at least 95%, at least 97%, at least 98%, or at least 99% identical to the reference albumin sequence defined by SEQ ID NO: 1. In various embodiments, the albumin amino acid sequence binds to the neonatal Fc receptor (FcRn), e.g., human FcRn. The albumin amino acid sequence may be a variant of wild-type HSA (e.g., as represented by SEQ ID NO: 1). In various embodiments, variants may have from one to twenty, or from one to ten amino acid deletions, substitutions, or insertions with respect to SEQ ID NO: 1. In some embodiments, the albumin amino acid sequence is any mammalian albumin amino acid sequence.

In some embodiments, the albumin amino acid sequence or domain is a fragment of full-length albumin, as represented by SEQ ID NO: 1. The term "fragment," when used in the context of albumin, refers to any fragment of full-length albumin or a variant thereof that extends the half-life of the CO-releasing group or complex comprising the same, to which it is conjugated, relative to the corresponding non-conjugated CO-releasing group or complex. Various modifications to the albumin sequence that enhance its ability to serve as a carrier are known, and such modifications can be employed. Exemplary modifications to the albumin amino acid sequence are described in US 8,748,380, US 10,233,228, and US 10,501,524.

A variant of a reference albumin sequence may share at least 50% sequence identity with the reference amino acid sequence, at least 55%, at least 60%, at least 65%, at least 70%, at least about 80%, at least 90%, at least 95%, at least 98% or at least 99% sequence identity. For example, a variant of a protein described herein may comprise an amino acid sequence that has at least 50% sequence identity with the reference amino acid sequence, at least 55%, at least 60%, at least 65%, at least 70%, at least about 80%, at least 90%, at least 95%, at least 98% or at least 99% sequence identity with the reference amino acid sequence.

Sequence identity is commonly defined with reference to the algorithm GAP (Wisconsin GCG package, Accelerys Inc, San Diego USA). GAP uses the homology alignment algorithm of Needleman & Wunsch, J. Mol. Biol. 48:443 (1970) to align two complete sequences that maximizes the number of matches and minimizes the number of gaps. Generally, default parameters are used, with a gap creation penalty = 12 and gap extension penalty = 4. Use of GAP may be preferred but other algorithms may be used, e.g. BLAST or BLAST 2.0 (which uses the method of Altschul et al. (1990) J. Mol. Biol. 215: 405-410), FASTA (which uses the similarity method of Pearson and Lipman (1988) PNAS USA 85: 2444-2448), or the local homology Smith-Waterman algorithm (Smith and Waterman (1981) J. Mol Biol. 147: 195-197), or the TBLASTN program, of Altschul et al. (1990) supra, generally employing default parameters. In particular, the psi-Blast algorithm may be used (Nucl. Acids Res. (1997) 25 3389-3402). Computerized implementations of these algorithms (GAP, BESTFIT, PASTA, and FASTA in the Wisconsin Genetics Software Package, Genetics Computer Group, 575 Science Dr., Madison, WI) are available and publicly available computer software may be used such as ClustalOmega (Söding, J. 2005, Bioinformatics 21, 951-960), T-coffee (Notredame et al. 2000, J. Mol. Biol. (2000) 302, 205-217), Kalign (Lassmann and Sonnhammer 2005, BMC Bioinformatics, 6(298)), Genomequest™ software (Gene-IT, Worcester MA USA) and MAFFT (Katoh and Standley 2013, Molecular Biology and Evolution, 30(4) 772-780 software. When using such software, the default parameters, e.g. for gap penalty and extension penalty, are preferably used. A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively.

Sequence comparisons are preferably made over the full-length of the relevant sequence described herein.

Suitable tumour-selective ligand proteins, such as human serum albumin, may be produced using standard recombinant techniques.

In some embodiments, the tumour-selective ligand is an antibody, or antigen-binding portion thereof. An antibody molecule is a polypeptide or protein comprising an antibody antigen-binding site. The term encompasses any immunoglobulin whether natural or partly or wholly synthetically produced. Antibody molecules can be isolated or obtained by purification from natural sources, or else obtained by genetic recombination, or by chemical synthesis, and that they may contain unnatural amino acids.

Antibodies suitable for use as tumour-selective ligands may include whole antibodies and fragments thereof. Fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) single-domain antibodies (sdAb) (also called nanobodies (Nb)) (Ward et al. (1989) Nature 341, 544-546; McCafferty et al., (1990) Nature, 348, 552-554; Holt et al. (2003) Trends in Biotechnology 21, 484-490), which consist of either a monomeric VH domain or a monomeric VL domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al. (1988) Science, 242, 423-426; Huston et al. (1988) PNAS USA, 85, 5879-5883); (viii) bispecific single chain Fv dimers (PCT/US92/09965) and (ix) "diabodies", multivalent or multispecific fragments constructed by gene fusion (WO94/13804; Holliger et al. (1993a), Proc. Natl. Acad. Sci. USA 90 6444-6448).

Fv, scFv, diabody, sdAb and other antibody molecules may be stabilized by the incorporation of disulphide bridges, for example linking the VH and VL domains (Reiter et al. (1996), Nature Biotech, 14, 1239-1245). Minibodies comprising a scFv joined to a CH3 domain may also be made (Hu et al. (1996), Cancer Res., 56(13):3055-61). Other examples of binding fragments are Fab', which differs from Fab fragments by the addition of a few residues at the carboxyl terminus of the heavy chain CH1 domain, including one or more cysteines from the antibody hinge region, and Fab'-SH, which is a Fab' fragment in which the cysteine residue(s) of the constant domains bear a free thiol group.

Antibodies suitable for use as described herein may bind specifically to a tumour antigen.

Other target-binding ligands and antibody mimetics may be employed as tumour-selective ligands, and which are known in the art. These include a single-domain antibody, a recombinant heavy-chain-only antibody (VHH), a single-chain antibody (scFv), a shark heavy-chain-only antibody (VNAR), a microprotein (e.g. cysteine knot protein, knottin), a DARPin, a Tetranectin, an Affibody; a Transbody, an Anticalin, an AdNectin, an Affilin, a Microbody, a phylomer, a stradobody, a maxibody, an evibody, a fynomer, an armadillo repeat protein, a Kunitz domain, an avimer, an atrimer, a probody, an immunobody, a triomab, a troybody, a pepbody, a vaccibody, a UniBody, and a DuoBody. Such ligands are described in the following references: US Patent Nos. or Patent Publication Nos. US 7,417,130, US 2004/132094, US 5,831,012, US 2004/023334, US 7,250,297, US 6,818,418, US 2004/209243, US 7,838,629, US 7,186,524, US 6,004,746, US 5,475,096, US 2004/146938, US 2004/157209, US 6,994,982, US 6,794,144, US 2010/239633, US 7,803,907, US 2010/119446, and/or US 7,166,697.

In some embodiments, tumour-selective ligands may be conjugated to the surface of nanoparticles. The nanoparticles may further encapsulate, or present on their surface, CO-releasing groups. In some embodiments, the CO-releasing groups may be conjugated to biodegradable nanomaterials. Exemplary nanoparticles include polymeric nanoparticles, which may include block co-polymers with PEG. For example, the terminus of PEG chains may have a functional group conjugation to the tumour-selective ligand and/or CO-releasing groups. Exemplary polymeric nanoparticle formats include those described in WO 2017/100597 and US 2018/0339024. Exemplary nanoparticles may include polymers such as poly(lactic-co-glycolic acid) (PLGA), poly(beta-amino ester) (PBAE), polycaprolactone (PCL), polyglycolic acid (PGA), polylactic acid (PLA), poly(acrylic acid) (PAA), poly-3-hydroxybutyrate (P3HB) and poly(hydroxybutyrate-co-hydroxyvalerate), including combinations thereof, and including block co-polymers of any of the foregoing with PEG. In other embodiments, the carbon monoxide releasing group(s) are conjugated to or encapsulated with inorganic materials. Nanomaterial delivery strategies for CORMS have been described [66].

The expression of one or more antigens (i.e. tumour antigens) may distinguish cancer cells from normal somatic cells in an individual. Normal somatic cells in an individual may not express the one or more antigens or may express them in a different manner, for example at lower levels, in different tissue and/or at a different developmental stage. Tumour antigens may therefore be used to target chimeric peptide exchange proteins specifically to cancer cells. Suitable tumour antigens expressed by cancer cells may include, for example, cancer-testis (CT) antigens encoded by cancer-germ line genes, such as MAGE-A1, MAGE-A2, MAGE- A3, MAGE-A4, MAGE-A5, MAGE-A6, MAGE-A7, MAGE-A8, MAGE-A9, MAGE-A10, MAGE-A11, MAGE-A12, GAGE-I, GAGE-2, GAGE-3, GAGE-4, GAGE-5, GAGE-6, GAGE-7, GAGE-8, BAGE-I, RAGE- 1, LB33/MUM-1, PRAME, NAG, MAGE-Xp2 (MAGE-B2), MAGE-Xp3 (MAGE-B3), MAGE-Xp4 (MAGE-B4), MAGE- C1/CT7, MAGE-C2, NY-ESO-I, LAGE-I, SSX-I, SSX-2(HOM-MEL-40), SSX-3, SSX-4, SSX-5, SCP-I and XAGE and immunogenic fragments thereof (Simpson et al. Nature Rev (2005) 5, 615-625, Gure et al., Clin Cancer Res (2005) 11, 8055-8062; Velazquez et al., Cancer Immun (2007) 7, 1 1 ; Andrade et al., Cancer Immun (2008) 8, 2; Tinguely et al., Cancer Science (2008); Napoletano et al., Am J of Obstet Gyn (2008) 198, 99 e91-97).

In some preferred embodiments, an antibody suitable for use as described herein may bind specifically to an immune checkpoint protein, such as PD-1 or CTLA4.

Small molecules suitable for use as tumour-selective ligands include small chemical molecules, for example non-polymeric organic compounds having a molecular weight of 900 Daltons or less. In preferred embodiments, a small molecule suitable for use as a tumour-selective ligand may have a molecular weight of 900 Daltons or less and accumulate selectively in tumour tissue. Suitable small molecule ligands include acetazolamide. Acetazolamide binds selectively to Carbonic anhydrase IX (CAIX), which is preferentially expressed in tumour tissue.

In some embodiments, the CO releasing group is conjugated to the tumour-selective ligand. Conjugation may be covalent i.e. the CO releasing group and tumour-selective ligand may be directly linked by a covalent bond, for example a dative covalent bond or coordinate bond.

Any suitable chemistry may be employed for the conjugation.

In some embodiments, a group which is present on the tumour-selective ligand may be able to bond to the CO releasing group directly, for example when the tumour-selective ligand is a peptide or a protein, an amino acid residue on the tumour-selective ligand may be able to bond directly to the CO releasing group in order to form a CO releasing conjugate. In other embodiments, a linker may be used to conjugate a CO releasing group to a tumour-selective ligand whereby the linker forms a covalent bond with both the CO releasing group and the tumour-selective ligand in order to form a CO releasing conjugate.

In some embodiments, a CO releasing group may be conjugated to the tumour-selective ligand by reacting a CO releasing molecule (CORM) with the tumour-selective ligand. The CORM may comprise a CO releasing group, for example a metal carbonyl complex. The metal atom of the CO releasing molecule (CORM) may be reacted with the tumour-selective ligand, thereby conjugating the CO releasing group of the CORM to the ligand. For example, the metal atom may form a covalent bond with an amino acid residue of the tumour-selective ligand, such as a histidine, cysteine, lysine, or methionine residue in the tumour-selective ligand protein.

In some embodiments, a metalation reaction may be employed. The tumour-selective ligand may be metalated with the metal atom of the CO releasing molecule (CORM), thereby conjugating the CO releasing group of the CORM to the ligand. The metalation reaction may be between the metal atom and a histidine residue on the tumour selective ligand. For example, the metal atom may form a covalent bond with the imidazole group of the histidine residue to conjugate the CO releasing group to the ligand.

The metal atom may be a transition metal. In some embodiments, the metalation reaction is between the ruthenium atom of a CORM comprising a ruthenium carbonyl complex, such as a Ru (CO)₂ complex, and a histidine present on the tumour-selective ligand protein. Suitable CORMs include CORM-3. For example, the ruthenium atom of a CORM comprising a ruthenium carbonyl complex may be covalently bonded with one or more histidine residue in human serum albumin. Preferably, the metalation reaction is between a [RuCl(κ²-H₂NCH₂CO₂)(CO)₃] complex (i.e. CORM-3) and a histidine present on human serum albumin.

In other embodiments, a linker is used to conjugate the CO releasing group to a tumour-selective ligand. For example, the CO releasing molecule may comprise a CO releasing group that is a metal carbonyl complex and the linker may comprise a first reactive moiety which is suitable for coordinating the metal carbonyl complex and a second reactive moiety which is suitable for forming a covalent bond with the tumour-selective ligand. In some embodiments, the linker is a PEG linker.

The first reactive moiety may for example be a metal ligand, such as a mono-, bi-, tri- or tetra-dentate metal ligand.

In some embodiments, the second reactive moiety may be, for example, a thiol reactive moiety suitable for conjugation with a thiol group on the tumour-selective ligand (i.e. thiol conjugation). The thiol reactive moiety may be any group suitable to form a covalent bond with a thiol group, such groups include haloacetyl groups, maleimides, aziridines, carbonylacrylic groups, halopyridazinediones, vinylsulfones, disulfides, and thiols. In some of these embodiments, the tumour-selective ligand is a protein and the thiol group is an amino acid residue, such as cysteine.

In other embodiments, the second reactive moiety may be a click reactive moiety suitable for undergoing a click reaction with a click handle on the tumour-selective ligand. The click reactive moiety may be any group suitable for undergoing a click reaction with a corresponding click handle on the tumour-selective ligand. Suitable click reaction combinations include copper (I)-catalyzed azide-alkyne cycloaddition (CuAAC); strain-promoted azide-alkyne cycloaddition (SPAAC); strain-promoted alkyne-nitrone cycloaddition (SPANC); alkene and azide [3+2] cycloaddition; and alkene tetrazine inverse-demand diels-alder. In some of these embodiments the tumour-selective ligand is a protein, in these embodiments suitable click handles include azides and alkynes, including strained and linear alkynes. Such suitable click handles may be introduced to the tumour-selective ligand protein by mutagenesis, for example by the introduction of an unnatural amino acid residue which comprises the click handle. Exemplary click handle containing amino acids include azidoalanine, azidophenylalanine, propargylglycine, propargylalanine, propynylproline. Alternatively, a click handle containing group may be conjugated to an amino acid on the tumour-selective ligand protein.

In some embodiments, the linker is first coordinated to the CORM, then the coordinated CORM is conjugated to the tumour selective ligand, for example by thiol conjugation or by click reaction. In other embodiments, the linker is first conjugated to the tumour selective ligand, for example by thiol conjugation or by click reaction, and then the CORM is coordinated by the linker-tumour-selective ligand conjugate.

In some embodiments, a CO releasing conjugate may comprise multiple CO releasing groups. For example, a CO releasing conjugate may comprise from 2 to about 20 CO releasing groups, such as about 5 to about 20, or about 10 to about 20, or about 2 to about 10, or about 2 to about 5 CO-releasing groups. The CO releasing conjugate may comprise 2, 3, 4, 5, 6, 7, 8, 9, 10, 11, 12, 13, 14, 15, 16 or more CO releasing groups.

The CO releasing groups may be conjugated to the same tumour-selective ligand. For example, two or more CO releasing groups may be conjugated to the tumour-selective ligand.

In some embodiments, a CO releasing conjugate may comprise human serum albumin with CO releasing groups conjugated to one or more histidine residues thereof. For example, 1, 2, 3, 4, 5, 6, 7, 8, 9, 10, 11, 12, 13 or more CO releasing groups may be conjugated, for example by dative covalent bonds, to histidine residues of human serum albumin. In some embodiments, from 8 to about 16 CO-releasing groups are conjugated to the human serum albumin.

A CO releasing conjugate as described herein may be produced by any convenient method. Suitable methods are available in the art (see for example [11]). For example, a CO releasing conjugate may be produced by reacting human serum albumin with a carbon dioxide releasing molecule (CORM). Examples of CORMs include transition metal CORMs, photoCORMs, enzyme triggered (ET)-CORMs, and organic CORMs. Transition metal CORMs are transition metal complexes primarily based on iron, molybdenum, ruthenium, cobalt, and rhenium. Ru(glycinate)CI(CO)₃ (CORM3) is an example of a transition metal CORM.

For example, a CO releasing conjugate may be produced by reacting human serum albumin with a CORM. In preferred embodiments, a CO releasing conjugate may be produced by reacting human serum albumin with Ru(glycinate)CI(CO)₃ (CORM3), such that one or more histidine residues of the human serum albumin are metalated with a ruthenium dicarbonyl group.

Suitable reaction conditions are well known in the art and include treating human serum albumin with about 50 equivalents of CORM-3 at room temperature for about 1 hour in PBS at physiological pH (e.g., pH 7.4).

While it is possible for a CO releasing conjugate as described herein to be administered to the individual alone, it is preferable to present the compound in a pharmaceutical composition or formulation. A pharmaceutical composition may comprise, in addition to the CO releasing conjugate as described herein, one or more pharmaceutically acceptable carriers, adjuvants, excipients, diluents, fillers, buffers, stabilisers, preservatives, lubricants, or other materials well-known to those skilled in the art. Such materials should be non-toxic and should not interfere with the efficacy of the active compound. The precise nature of the carrier or other material will depend on the route of administration, which may be by bolus, infusion, injection or any other suitable route, as discussed below. Suitable materials will be sterile and pyrogen free, with a suitable isotonicity and stability. Examples include sterile saline (e.g. 0.9% NaCl), water, dextrose, glycerol, ethanol or the like or combinations thereof. The composition may further contain auxiliary substances such as wetting agents, emulsifying agents, pH buffering agents or the like.

Suitable carriers, excipients, etc. can be found in standard pharmaceutical texts, for example, Remington's Pharmaceutical Sciences, 18th edition, Mack Publishing Company, Easton, Pa., 1990.

The term "pharmaceutically acceptable" as used herein pertains to compounds, materials, compositions, and/or dosage forms which are, within the scope of sound medical judgement, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

The formulations may conveniently be presented in unit dosage form and may be prepared by any methods well-known in the art of pharmacy. Such methods include the step of bringing into association the active compound with the carrier which constitutes one or more accessory ingredients. In general, the formulations are prepared by uniformly and intimately bringing into association the active compound with liquid carriers or finely divided solid carriers or both, and then if necessary shaping the product.

Formulations may be in the form of liquids, solutions, suspensions, emulsions, elixirs, syrups, tablets, lozenges, granules, powders, capsules, cachets, pills, ampoules, suppositories, pessaries, ointments, gels, pastes, creams, sprays, mists, foams, lotions, oils, boluses, electuaries, or aerosols.

A CO releasing conjugate as described herein or pharmaceutical compositions comprising the CO releasing conjugate may be administered to a subject by any convenient route of administration, whether systemically/peripherally or at the site of desired action, including parenteral, for example, by injection, including subcutaneous, intradermal, intramuscular, intravenous, intraarterial, intratumoral, intracardiac, intrathecal, intraspinal, intracapsular, subcapsular, intraorbital, intraperitoneal, intratracheal, subcuticular, intraarticular, subarachnoid, and intrasternal; by implant of a depot, for example, subcutaneously or intramuscularly. Administration is preferably either intravenous or at the site of desired action (e.g., intratumoral injection). Intravenous is preferable for many indications, such as colorectal cancer, although administration at the site of desired action may be preferable for some indications, such as glioblastoma or bladder cancer.

The pharmaceutical compositions comprising a compound described herein may be formulated in a dosage unit formulation that is appropriate for the intended route of administration.

Formulations suitable for parenteral administration (e.g. by injection, including cutaneous, subcutaneous, intramuscular, intravenous and intradermal), include aqueous and non-aqueous isotonic, pyrogen-free, sterile injection solutions which may contain anti-oxidants, buffers, preservatives, stabilisers, bacteriostats, and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. Examples of suitable isotonic vehicles for use in such formulations include Sodium Chloride Injection, Ringer's Solution, or Lactated Ringer's Injection. Typically, the concentration of the active compound in the solution is from about 1 ng/ml to about 10 µg/ml, for example, from about 10 ng/ml to about 1 µg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets. Formulations may be in the form of liposomes or other microparticulate systems which are designed to target the active compound to macrophages or adipose tissue.

Optionally, other therapeutic or prophylactic agents may be included in the pharmaceutical composition or formulation.

CO releasing conjugates as described herein may be useful in reducing immunosuppression in tumour tissue, for example by reducing expression of immune checkpoint proteins and/or the amount of tumour associated macrophages (TAMs) in the tumour tissue. For example, CO releasing conjugates may be useful in the treatment of immunosuppressive cancers and/or for potentiating cancer immunotherapy.

Cancer is characterised by the abnormal proliferation of malignant cancer cells. An immunosuppressive cancer is a cancer in which the immune system of the individual is suppressed in tumour tissue i.e. within the tumour and its microenvironment (e.g. the blood and lymphatic vessels, extracellular matrix, host cells, such as fibroblasts, neuroendocrine (NE) cells, adipose cells, and immune-inflammatory cells, and secreted factors that surround the tumour). This immune suppression reduces or prevents host immune responses against cancer cells.

An immunosuppressive cancer may be characterised by tumours that inhibit, reduce or prevent an immune response against cancer cells in the tumour. For example, the microenvironment of the tumour may be immunosuppressive and T-cell inhibitory proteins and/or T-cell inhibitory cells that suppress an immune response may be present in the tumour microenvironment.

In some embodiments, suppression of the immune response by a tumour may result from the inhibition of T cells i.e. T cells in the tumour and/or its microenvironment may display reduced activity.

The inhibition of T cells may be mediated by one or more immune checkpoint proteins, such as PD-1 and CTLA-4. Immune checkpoint proteins are regulators of the immune system and are crucial for self-tolerance, which prevents the immune system from attacking cells indiscriminately. However, the expression of immune checkpoint proteins by malignant cells dysregulates the anti-tumour immunity and promotes the growth and expansion of cancer cells [13].

An immunosuppressive cancer may be characterised by the expression of immune checkpoint proteins or an increased level of expression or activity of immune checkpoint proteins compared with non-cancer cells. The expression level or activity of checkpoint proteins may be determined by common methods known in the art such as mass spectrometry, flow cytometry, qPCR, and RNA sequencing.

Immune checkpoint proteins may include PD-1. Programmed cell death protein 1 (Gene ID 5133; also known as PD-1 or CD279 (cluster of differentiation 279)) is a T-cell inhibitory protein found on the surface of T cells. PD-1 has a role in regulating the immune system's response by down-regulating the immune system and promoting self-tolerance by suppressing T cell activity. However, this may prevent the immune system from killing cancer cells. PD-1 may have the reference amino acid sequence of NCBI database entry NP_005009.2 and may be encoded by the reference nucleotide sequence of NCBI database entry NM_005018.3.

The interaction of PD-1 on T cells with its ligands PD- L1 or PD-L2 on cancer cells of immunosuppressive tumours (as well as on dendritic cells and macrophages) can inhibit the activity of the T cells and allow cancer cells to evade host immune responses. Reducing the expression of PD-1 on T cells using a CO releasing conjugate as described herein may reduce or inhibit the PD-1/PD-L1 and/or PD-1/PD-L2 interaction and thereby prevent or reduce the evasion of the host immune system by the cancer cells.

PD-1 quantification, if desired, may be performed by any convenient method, for example immunofluorescence detection of the mean fluorescence intensity per pixel for each isolated T cell or tumour cell using the ARIOL system [61], by flow cytometry or by western blot.

Immune checkpoint proteins may include CTLA-4. Cytotoxic T-lymphocyte associated antigen 4 (Gene ID No: 1493; also known as CTLA4, CTLA-4, or CD152 (cluster of differentiation 152)) is a T-cell inhibitory protein that functions as an immune checkpoint and downregulates immune responses. CTLA-4 is constitutively expressed in regulatory T cells and upregulated in conventional T cells after activation. Upregulation of CTLA-4 in cancers (e.g., immunosuppressive cancers) may prevent the immune system from killing cancer cells. CTLA-4 may have the reference amino acid sequence of NCBI database entry NP_005205.2 and may be encoded by the reference nucleotide sequence of NCBI database entry NM_005214.5.

The interaction of CTLA-4 on T cells with its ligands CD80 and CD86, on cancer cells may lead to the inhibition of T cell activity and cause the cancer cells to evade host immune responses. Reducing the expression of CTLA-4 on T cells using a CO releasing conjugate as described herein may reduce or prevent the CTLA-4/ligand interaction and thereby prevent or reduce the inhibition of T cells and the evasion of the host immune system by the cancer cells.

CTLA-4 quantification, if desired, may be performed by any convenient method, for example immunofluorescence detection of the mean fluorescence intensity per pixel for each isolated T cell or tumour cell using the ARIOL system [61], by flow cytometry or by western blot.

In other embodiments, an immunosuppressive cancer may be characterised by the presence of T-cell inhibitory cells, such as tumour associated macrophages (TAMs) and/or TAM progenitors or an increased number of TAMs and/or TAM progenitors compared with non-cancer cells.

The presence of T-cell inhibitory cells, such as regulatory T-cells, tumour associated macrophages (TAMs), and their progenitors, in the tumour microenvironment may cause or contribute to T-cell inhibition and immunosuppression. For example, tumour associated macrophages (TAMs) populate the tumour microenvironment and commonly display an M2 macrophage-like phenotype. TAMs suppress anti-tumour immune responses, and promote tumour development, involving functions such as the sustained accumulation of Treg cells and dysregulation of the vasculature due to the expression of chemokines and amino acid-degrading enzymes, such as arginase 1 and indoleamine-2, 3-dioxygenase (IDO) [13]. The accumulation of TAMs in the microenvironment of a tumour in an individual is associated with resistance to cancer therapies and poor clinical prognosis.

The accumulation of TAMs in the microenvironment of a tumour in an individual may lead to the inhibition of T cell activity and the suppression of immune responses to the tumour. Reducing the amount of TAMS in the microenvironment of a tumour using a CO releasing conjugate as described herein may prevent or reduce the inhibition of T cells and the evasion of the host immune system by the cancer cells.

An immunosuppressive cancer may be of any cancer type, including solid cancers such as sarcomas, carcinomas, and lymphomas, including skin cancer, melanoma, bladder cancer, brain cancer, breast cancer, uterus cancer, oral cancer, ovary cancer, prostate cancer, lung cancer, colorectal cancer, cervical cancer, liver cancer, head and neck cancer, oesophageal cancer, pancreas cancer, renal cancer, adrenal cancer, stomach cancer, testicular cancer, cancer of the gall bladder and biliary tracts, thyroid cancer, thymus cancer, and bone cancer.

An immunosuppressive cancer can comprise any one or more of the following: acute lymphocytic leukemia (ALL), acute myeloid leukemia (AML), adrenocortical cancer, anal cancer, bladder cancer, blood cancer, bone cancer, brain tumor, breast cancer, cancer of the female genital system, cancer of the male genital system, central nervous system lymphoma, cervical cancer, childhood rhabdomyosarcoma, childhood sarcoma, chronic lymphocytic leukemia (CLL), chronic myeloid leukemia (CML), colon and rectal cancer, colon cancer, endometrial cancer, endometrial sarcoma, esophageal cancer, eye cancer, gallbladder cancer, gastric cancer, gastrointestinal tract cancer, hairy cell leukemia, head and neck cancer, hepatocellular cancer, Hodgkin's disease, hypopharyngeal cancer, Kaposi's sarcoma, kidney cancer, laryngeal cancer, leukemia, leukemia, liver cancer, lung cancer, malignant fibrous histiocytoma, malignant thymoma, melanoma, mesothelioma, multiple myeloma, myeloma, nasal cavity and paranasal sinus cancer, nasopharyngeal cancer, nervous system cancer, neuroblastoma, non-Hodgkin's lymphoma, oral cavity cancer, oropharyngeal cancer, osteosarcoma, ovarian cancer, pancreatic cancer, parathyroid cancer, penile cancer, pharyngeal cancer, pituitary tumor, plasma cell neoplasm, primary CNS lymphoma, prostate cancer, rectal cancer, respiratory system, retinoblastoma, salivary gland cancer, skin cancer, small intestine cancer, soft tissue sarcoma, stomach cancer, stomach cancer, testicular cancer, thyroid cancer, urinary system cancer, uterine sarcoma, vaginal cancer, vascular system, Waldenstrom's macroglobulinemia and Wilms' tumor.

Cancers may be of a particular type. Examples of types of cancer include astrocytoma, carcinoma (e.g. adenocarcinoma, hepatocellular carcinoma, medullary carcinoma, papillary carcinoma, squamous cell carcinoma), glioma, lymphoma, medulloblastoma, melanoma, myeloma, meningioma, neuroblastoma, sarcoma (e.g. angiosarcoma, chrondrosarcoma, osteosarcoma).

In some embodiments, the cancer is a primary tumour, or is metastatic cancer. "Metastasis" refers to the spread of cancer from a primary site to other places in the body. Cancer cells can break away from a primary tumour, penetrate into lymphatic and blood vessels, circulate through the bloodstream, and grow in a distant focus (metastasize) in normal tissues elsewhere in the body. Metastasis can be local or distant. In various embodiments, the primary or metastatic cancer is melanoma, lung cancer, kidney cancer, prostate cancer, cervical cancer, colorectal cancer, pancreatic cancer, ovarian cancer, urothelial cancer, gastric cancer, head and neck cancer, glioblastoma, head and neck squamous cell carcinoma (HNSCC), non-small cell lung carcinoma (NSCLC), small cell lung cancer (SCLC), bladder cancer, and prostate cancer (e.g. hormone-refractory). In some embodiments, the cancer is progressive, locally advanced, or metastatic carcinoma. In some embodiments, the cancer is metastatic melanoma, and may be recurrent. In some embodiments, the metastatic melanoma is stage III or IV. The metastasis may be regional or distant.

In some embodiments, immunosuppressive cancer may be of a cancer type which has received approval for treatment with an immune checkpoint inhibitor, for example a cancer type which has received approval for treatment with a CTLA-4 inhibitor and/or PD-1 inhibitor (see for example [20] to [60]). In some preferred embodiments, immunosuppressive cancer may include melanoma, squamous cell carcinoma, colorectal cancer, lung cancer, including non-small cell lung cancer (NSCLC), renal carcinoma, and urothelial carcinoma.

Immunosuppressive tumours may be characterised by an increased tumour mutation burden (TMB) ([52-55]) and/or increased numbers of tumour-infiltrating lymphocytes ([56, 57]) relative to other cancers.

An immunosuppressive cancer may be identified in an individual using standard diagnostic criteria. Examples of such clinical standards can be found in textbooks of medicine such as Harrison's Principles of Internal Medicine, 15th Ed., Fauci AS et al., eds., McGraw-Hill, New York, 2001.

Administration *in vivo* and parts related to the treatment of a subject is not part of the invention.

The term "treatment", as used herein in the context of treating a condition, pertains generally to treatment and therapy in which some desired therapeutic effect is achieved, for example, the inhibition of the progress of the condition, and includes a reduction in the rate of progress, a halt in the rate of progress and amelioration of the condition, and cure of the condition. In various examples, treatment can result in an increase an overall survival, increase in progression-free interval, or result in stable disease or tumour regression, or result in a designation of cancer-free.

The individual may have been previously identified as having an immunosuppressive cancer or be at risk of developing an immunosuppressive cancer. A method may comprise identifying the patient as having or being at risk of developing an immunosuppressive cancer before administration.

An individual suitable for treatment as described above may be a mammal, such as a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orang-utan, gibbon), or a human. Preferably, the individual is a human. Non-human mammals, especially mammals that are conventionally used as models for demonstrating therapeutic efficacy in humans (e.g. murine, primate, porcine, canine, or leporid) may be employed.

Treatment may be any treatment and therapy, whether of a human or an animal (e.g. in veterinary applications), in which some desired therapeutic effect is achieved, for example, the inhibition or delay of the progress of the condition, and includes a reduction in the rate of progress, a halt in the rate of progress, amelioration of the condition, cure or remission (whether partial or total) of the condition, preventing, delaying, abating or arresting one or more symptoms and/or signs of the condition or prolonging survival of a subject or patient beyond that expected in the absence of treatment.

Treatment as a prophylactic measure (i.e. prophylaxis) is also included. For example, an individual susceptible to or at risk of the occurrence or re-occurrence of immunosuppressive cancer may be treated as described herein. Such treatment may prevent or delay the occurrence or re-occurrence of cancer in the individual. In particular, treatment may include inhibiting cancer growth, including complete cancer remission, and/or inhibiting cancer metastasis. Cancer growth generally refers to any one of a number of indices that indicate change within the cancer to a more developed form. Thus, indices for measuring an inhibition of cancer growth include a decrease in cancer cell survival, a decrease in tumour volume or morphology (for example, as determined using computed tomographic (CT), sonography, or other imaging method), a delayed tumour growth, a destruction of tumour vasculature, improved performance in delayed hypersensitivity skin test, an increase in the cytolytic activity of cancer cells, and a decrease in levels of tumour-specific antigens.

In some embodiments, an individual suitable for treatment as described herein may have minimal residual disease (MRD) after an initial cancer treatment.

A CO releasing conjugate as described herein may be administered as described herein in a therapeutically-effective amount. The term "therapeutically-effective amount" as used herein, pertains to that amount of an active compound, or a combination, material, composition or dosage form comprising an active compound, which is effective for producing some desired therapeutic effect, commensurate with a reasonable benefit/risk ratio.

The appropriate dosage of a CO releasing conjugate as described herein may vary from individual to individual. Determining the optimal dosage will generally involve the balancing of the level of therapeutic benefit against any risk or deleterious side effects of the administration. The selected dosage level will depend on a variety of factors including, but not limited to, the route of administration, the time of administration, the rate of excretion of the active compound, other drugs, compounds, and/or materials used in combination, and the age, sex, weight, condition, general health, and prior medical history of the individual. The amount of active compounds and route of administration will ultimately be at the discretion of the physician, although generally the dosage will be to achieve therapeutic plasma concentrations of the active compound without causing substantial harmful or deleterious side-effects.

In general, a suitable dose of the active compound is in the range of about 100 µg to about 400 mg per kilogram body weight of the subject per day, preferably 200 µg to about 200 mg per kilogram body weight of the subject per day. Where the active compound is a salt, an ester, prodrug, or the like, the amount administered is calculated on the basis of the parent compound and so the actual weight to be used is increased proportionately.

Administration in vivo can be effected in one dose, continuously or intermittently (e.g., in divided doses at appropriate intervals).

Methods of determining the most effective means and dosage of administration are well known in the art and will vary with the formulation used for therapy, the purpose of the therapy, the target cell being treated, and the subject being treated. Single or multiple administrations can be carried out with the dose level and pattern being selected by the physician.

Multiple doses of the compound comprising a CO releasing conjugate as described herein may be administered, for example 2, 3, 4, 5 or more than 5 doses may be administered. The administration of the compound comprising a CO releasing conjugate as described herein may continue for sustained periods of time. For example treatment with the compound comprising a CO releasing conjugate as described herein may be continued for at least 1 week, at least 2 weeks, at least 3 weeks, at least 4 weeks, at least 1 month or at least 2 months, or at least 3 months. Treatment with the compound comprising a CO releasing conjugate as described herein may be continued for as long as is necessary to reduce immunosuppression. In some embodiments, the CO releasing conjugate is administered once or twice per week or once or twice per month (e.g., for two to twelve months or from two to eight months). In some embodiments, the CO releasing conjugate is administered intravenously.

The compound comprising a CO releasing conjugate as described herein may be administered alone or in combination with other treatments, either simultaneously or sequentially dependent upon the individual circumstances. For example, a compound comprising a CO releasing conjugate as described herein as described herein may be administered in combination with one or more additional active compounds.

The compound comprising a CO releasing conjugate as described herein may be administered in combination with one or more other cancer therapies, such as immunotherapy (e.g. checkpoint inhibitor therapy, T cell co-stimulator therapy, or cytokine therapy), cell therapy, cytotoxic chemotherapy or radiotherapy.

For example, the CORM-conjugate may be administered in combination with a cytokine therapy, T cell costimulator therapy, or an immune checkpoint inhibitor. Cytokine therapy activates the immune system of cancer patients. Interferon alpha (IFNα) is approved for adjuvant treatment of completely resected high-risk melanoma patients and several refractory malignancies. High-dose interleukin-2 (IL-2) is approved for treatment of metastatic renal cell cancer and melanoma. An immune checkpoint inhibitor is a molecule or compound that inhibits the ligand binding or activity of an immune checkpoint protein, such as CTLA-4 and PD-1. Suitable immune checkpoint inhibitors include anti-CTLA-4 antibodies, such as ipilimumab (BMS), and tremelimumab (AZ); anti-PD-1 antibodies, such as nivolumab (BMS), and pembrolizumab (Merck); and anti-PD-L1 antibodies, such as durvalumab (AZ), atezolizumab (Genentech/Roche), and avelumab (Merck Kga/Pfizer). T cell costimulation therapy can include agonists against CD28 or OX40 agonists, for example.

When the therapeutic agents are used in combination with additional therapeutic agents, the compounds may be administered either sequentially or simultaneously by any convenient route. When a therapeutic agent is used in combination with an additional therapeutic agent active against the same disease, the dose of each agent in the combination may differ from that when the therapeutic agents are used alone. Appropriate doses will be readily appreciated by those skilled in the art.

Administration of therapeutic agents, as described herein, can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell being treated, and the subject being treated. Single or multiple administrations can be carried out with the dose level and pattern being selected by the treating physician.

In various embodiments, the conjugate is administered as a regimen with a cancer immunotherapy. In various embodiments, the conjugate is administered to a patient before, during, and/or after a cancer immunotherapy regimen. For example, the cancer immunotherapy may be an antibody or antigen-binding portion thereof that blocks an immune checkpoint molecule, e.g., a molecule selected from B7-H3, B7-H4, BTLA, CD160, CTLA4, KIR, LAG3, PD-1, PD-L1, PD-L2, TIM3, LAG3, and TIGIT. In some embodiments, the cancer immunotherapy comprises an agonist of an immune co-stimulatory molecule, e.g., a molecule selected from 4-1BB, CD27, CD28, CD40, CD137, GITR, ICOS, OX40, TMIGD2, and TNFRSF25. In some embodiments, the cancer immunotherapy is a TLR9 agonist. In embodiments, the agonist is an antibody or antigen-binding portion thereof (including bi-specific antibodies) or a small molecule. Exemplary agents include ipilimumab, pembrolizumab, tremelimumab, nivolumab, and pidilizumab.

In some embodiments, the cancer immunotherapy is an adoptive cell therapy, which can include T cell adoptive cell therapy, including a recombinant T cell that expresses a chimeric antigen receptor (CAR) which recognizes and binds a tumour antigen. In some embodiments, the CAR-T is targeted to CD19.

In other embodiments, the immunotherapy comprises bone marrow transplant or administration of hematopoietic stem cells.

In various embodiments, the conjugate is administered to the cancer patient in connection with a regimen of an anti-PD1, anti-PDL1 and/or anti-CTLA4 antibody.

In various embodiments, the therapeutic regimen includes administration of the conjugate for one to four weeks before initiating the immunotherapy regimen (e.g., immune checkpoint inhibitor therapy or adoptive cell therapy), so as to condition the patient for cancer immunotherapy. In still other embodiments, the therapeutic regimen includes administration of the conjugate for one or more weeks or months after the completion of a cancer immunotherapy regimen, so as to improve the efficacy and/or duration of the effect. In some embodiments, the conjugate and immunotherapy are administered in a regimen that includes concomitant therapy.

In various embodiments, the patient was previously unresponsive to, only partially responsive to, or had become resistant to, a checkpoint inhibitor therapy, such as anti-CTLA-4, anti-PD-1, or anti-PD-L1 and/or anti-PD-L2 agent. For example, the patient may have received a prior regimen of such immune checkpoint inhibitor therapy, or other immunotherapy described herein, but was unresponsive or only partially responsive to said therapy. These refractory patients can benefit from the CO releasing group conjugate, optionally in connection with another immunotherapy as described herein.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

As used herein, the term "about" means ±10% of an associated number, unless the context requires otherwise.

### Experimental

Gaseous mediators are small molecules with multiple physiological roles and capable of conveying instructive cues to immune cells. In this experimental study, we show that an artificial albumin metalloprotein (rHSA-CORM) is capable of specifically delivering significant amounts of CO to tumours where it restrains progression. In situ, CO reduces the expression of immune checkpoint molecules and the number of tumour-associated macrophages that facilitate immune evasion, unleashing the production of anti-tumour immune responses. Notably, rHSA-CORM and immune checkpoint blockade (ICB) synergise to eliminate tumours, prompting immunological memory. These results highlight the potential of CORMs and protein conjugates thereof as therapeutic regimens in cancer treatment.

Immunotherapy, a treatment strategy that harnesses the immune system to specifically target tumour cells, has completely changed the outlook for cancer patients by becoming the standard of care for various types of cancer. However, and despite great achievements made over the past decades, immunotherapeutics are not effective in all patients, as cancer cells outmanoeuvre immune recognition and destruction [5]. Thus, new tactics in promoting anti-tumour immune responses are needed. CO is a low molecular weight gas with biological functions spanning from immunomodulation to cytoprotection. Naturally present in the environment, it selectively reacts with transition metals present in certain proteins that transduce its physiological activity. Previous research on CO biology has primarily focused on its direct effects in vitro. The role of CO in cancer immunity is virtually unknown. Inasmuch as the exposure to high concentrations of CO is severely poisonous, decaging systems such as CO-releasing molecules (CORMs) safely provide higher therapeutic doses. However, CORMs have no tissue or cell selectivity and therefore require carriers that enable a more controlled delivery at specific tissues. Herein, we describe a therapeutic strategy that couples the anti-tumour potential of CORMs (e.g., CORM-3) with the tumour-targeting capacity of recombinant human serum albumin (rHSA) [14] into a single agent. Using in vivo cancer models, we observed that rHSA-CORM treatments were effective in attenuating tumour growth and in prolonging overall survival of mice in an immune-dependent fashion, showing its promise as an anticancer immunotherapeutic agent. Importantly, CO-treated mice exhibited a reduction in the expression of immune-checkpoint markers CTLA-4 and PD-1 and in the frequency of tumour associated macrophages (TAMs) that facilitate immune suppression. Combination therapy with ICB enabled complete tumour regression and immunological memory. Remarkably, these results indicate that immune evasion in cancer could be reversed by boosting the efficacy of currently implemented therapies with the administration of CORMs.

### Materials and Methods

### Conjugation and purification

Reaction of recombinant human serum albumin (rHSA) with 50 equivalents of CORM-3 (Sigma) in phosphate-buffered saline (PBS) at pH 7.4 for 1 hour at room temperature produced a single peak corresponding to the successful metalation of rHSA as detected by liquid chromatography MS (LC-MS).

### Animal studies and cell isolation of tumours

On day 0, 5×10⁵ CT26 cells (colon carcinoma) were injected subcutaneously into the abdomens of BALB/c (or NSG) mice with 50 µl of a 1:1 mixture of Dulbecco's modified Eagle medium with Matrigel (Corning). The same protocol was applied in C57BL/c mice using 1x10⁸ MC38 (colon adenocarcinoma) cells instead. rHSA-CORM and rHSA-DMSO were administered daily once tumour volumes reached around 100 mm³, by intratumoural (i.t.) injection in 50 µl PBS (30 mg kg⁻¹). Mice were euthanized when tumour volume reached 1 cm³.

After the induction of ectopic tumours, BALB/c mice were treated daily with CO-conjugates until analysis. At day 13, tumours were excised, minced and digested using a mix of collagenase I, collagenase IV (Worthington) and DNase I (Roche) in a shaker for 20 min, 250 r.p.m. at 37 °C. After digestion, samples were passed through a 100-µm cell strainer, and resuspended in cold complete RPMI 1640 medium supplemented with 10 mM HEPES buffer, 1 mM sodium pyruvate, 50 µM 2-mercaptoethanol, 100 U/ml penicillin and 100 µg/ml streptomycin and complemented with 1% non-essential amino acids (NEAA), 1% GlutaMAX supplement and 10% heat-inactivated FBS (HI FBS). Cells were resuspended in PBS with 2% FBS and 1 mM EDTA and stained for CD45-APC for 15 min at RT, followed by a 15 min staining with MojoSort^{™} Mouse anti-APC Nanobeads (BioLegend) at RT. Stainings and samples were maintained at RT to prevent neutrophil disruption. 3 cycles of positive selection were performed with a magnetic stand and cells were stained for the evaluation of different markers for flow cytometric analysis.

### CO level determination

CO levels were determined in tissues and blood using the method described by Vreman and co-workers [18]. CO release was assessed in Bone-marrow-derived macrophage cells (BMDMs) and colorectal carcinoma cells (CT26). CO biodistribution in mice and carboxyhaemoglobin (CO-Hb) levels in circulation were assessed 30 minutes after an i.v. injection 3 mg kg⁻¹ of rHSA-CORM-3 or rHSA alone.

### Live-imaging of CO release in CT26 cells and BMDMs

Detection of COP-1 using confocal microscopy as readout of CO-release was performed following previously described methods [9]. Briefly, images were obtained using a Zeiss LSM 880 confocal Laser Point-Scanning Microscope with a 40X water objective lens and a numerical aperture of 1.3. COP-1 was excited using a 488 nm Argon Laser and fluorescence was detected at a wavelength range of 500-550 nm. 1.5x10⁴ CT26 cells or BMDMs were seeded in 8-chambered #1.0 Borosilicate Coverglass (Lab-Tek) plates in 200uL, two days before the experiment. The cells were incubated with either 1.5 µM rHSA-CORM or rHSA-DMSO for 30 minutes and washed 3x with PBS before acquisition. A control image was taken before and after the addition of 1 µM of COP-1 at the microscope. Images were taken every 5 minutes for up to 60 minutes after the addition of the probe. Image acquisition was performed in a Z stack to ensure cells would be fully imaged along the Z-axis. Acquisition was performed under 37°C and with 5% CO₂.

### Tumour size and survival analysis

Tumour size was measured every day with a digital caliper using the following formula: volume = length × width × width × 0.5. Mice were monitored after inoculation and survival rates were calculated. Tumour growth results were analysed by one-way ANOVA, if significant (95% confidence interval). Comparisons of the survival of tumour-bearing mice were performed using the log-rank Mantel-Cox test (95% confidence interval).

### Mass spectrometry

A 20 µL of BSA and BSA-Rull(CO)₂ samples were buffer exchanged into 200 mM ammonium acetate buffer (pH 7) using Micro Bio-Spin 6 columns (Bio-Rad). Mass spectra were acquired on a high-mass Q-TOF-type instrument Xevo G2-S (Waters, Manchester, UK). Mass spectrometry experiments were performed at a capillary voltage of 1500 V, cone voltage of 200 V and source offset voltage of 150 V. Spectra were processed using MassLynx V4.1 (Waters).

### Cell culture

CT26 cells (ATCC) were routinely grown in a humidified incubator at 37 °C under 5% CO₂ and split twice a week before reaching confluence, using TrypLE Express. CT26 cells were grown on DMEM medium supplemented with 10% heat-inactivated FBS, 1% Glutamax, 1% Sodium Pyruvate, 10 mM HEPES, 10 mM NEAA, 200 units/mL penicillin and 200 µg/mL streptomycin. All reagents were bought from Gibco, Life Technologies (USA), unless otherwise stated.

### Assessment of immune response

Total cells were harvested from the tumours of 8 to 14 weeks old BALB/c mice, imported from Charles River. Excised tumours were minced and digested using a mix of collagenase I, collagenase IV (Worthington) and Dnase I (Roche) in a shaker for 20 min, 250 r.p.m. at 37 °C. After digestion, samples were passed through a 100-µm cell strainer, and resuspended in cold complete RPMI 1640 medium, supplemented with 10 mM HEPES buffer, 1 mM sodium pyruvate, 50 µM 2-mercaptoethanol, 100 U/ml penicillin and 100 µg/ml streptomycin and complemented with 1% non-essential amino acids (NEAA), 1% GlutaMAX supplement and 10% heat-inactivated FBS (HI FBS). Cells were resuspended in PBS with 2% FBS and 1 mM EDTA and stained for extracellular markers for 45 min at 4 °C. Cell suspensions were then fixed, permeabilized and stained for intracellular markers using the eBioscience Foxp3 Transcription Factor Staining Buffer Set from ThermoFisher Scientific. Samples were analysed in a BD LSRFortessa flow cytometer equipped with a BD FACSDiva software and data were analysed in FlowJo v.10 software. Antibodies (BioLegend) used in colon carcinoma experiments were: CD45-BV510 (30-F11), CD3-BV711 (17A2), CD49b-FITC (DX5), CD4-BV605 (RM4-5), CD8-PECy7 (53-6.7), CD152-PECy7 (CTLA-4, UC10-4B9), CD279-PE (PD-1, 29F.1A12), TNFα-PE (MP6-XT22), IFNγ-PerCP/Cy5.5 (XMG1.2), I-A/I-E-PE (MHC-II, M5/114.15.2), F4/80-PECy7 (BM8), Ly6G-BV605 (1A8), Ly6C-FITC (HK1.4), CD11c-BV711 (N418), TER119-APC, CD19-APC (6D5), Ki-67-BV605 (16A8), CD274-BV421 (B7-H1, PD-L1, 10F.9G2), CD206-PerCP/Cy5.5 (C068C2). Antibodies (eBioscience) used were: iNOS-PE (CXNFT) and ICAM-1-FITC (YN1/1.7.4). Fixable Viability Dye eFluor 780 (eBioscience) was used to exclude dead cells. Animals were maintained according to protocols approved by the Direção Geral de Veterinária and Instituto de Medicina Molecular João Lobo Antunes ethical committee.

### Macrophage depletion analysis

Following the aforementioned protocol of CT26 tumour induction *in vivo,* macrophage depletion was performed by daily i.v. injections of 600µg/kg of anti-CCR2 antibody (MC-21) and twice-a-week i.v. injections of clodronate liposomes, for the depletion of macrophages, starting at day 9 after CT26 cell s.c. injection. At day 16 mice were sacrificed, cells were isolated and evaluated through flow cytometry. Flow cytometry analysis was performed as previously mentioned.

### Bone marrow-derived macrophage (BMDM) culture systems

BM cells were isolated from the femurs of 8-week old C57BL/c mice and placed in 6-well plates containing 800µL RPMI 1640 medium (per well) supplemented with 10% heat-inactivated FBS (HI FBS), 10 mM HEPES buffer, 1 mM sodium pyruvate, 20 U/ml penicillin and 20 µg/ml streptomycin and complemented 1% GlutaMAX. We further added 200 µL of the supernatants of M-CSF-producing L929 fibroblasts to each well. Cells were maintained in a humidified incubator at 37 °C and 5% CO₂ for 6 days for quasi-total macrophage differentiation. rHSA-CORM or rHSA-DMSO conjugates were added 24h after seeding and re-added at day 3 together with 250µL of L929 supernatant, per well. At day 6, cells were incubated with 2mM EDTA in PBS for approx. 10 min, and mechanically removed by scratching and re-seeded in 12 well plates. Macrophage polarisation was achieved by culturing BMDM for 24h in the following conditions: M0: 20% L929 (or 10 ng/mL M-CSF); M1: 10 ng/mL LPS + 10 ng/mL IFNγ; M2a: 10 ng/mL IL-4 + 10 ng/mL IL-13; M2c: 10 ng/mL IL-10 + 0.5 ng/mL TGF-β. Cells were harvested from plates as aforementioned and analysed by flow cytometry in a BD Fortessa instrument.

### Results

### Mass Spectometry

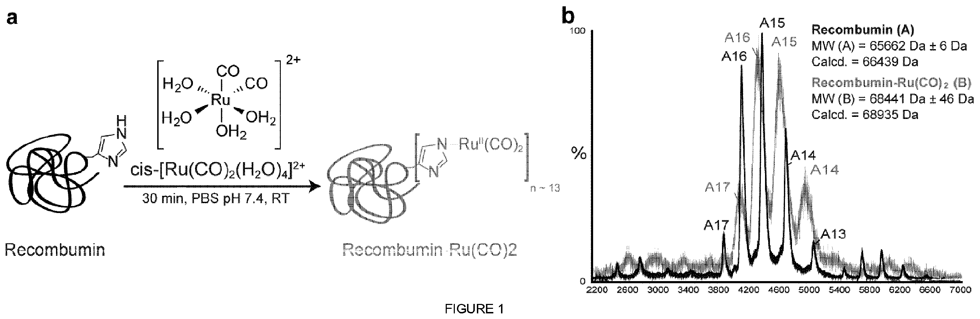

A schematic representation of the conjugation strategy and the mass spectra of native rHSA (Recombumin) and rHSA-CORM (Recombumin-Ru(CO)2) are presented in Figure 1a and 1b, respectively.

### CO release and distribution

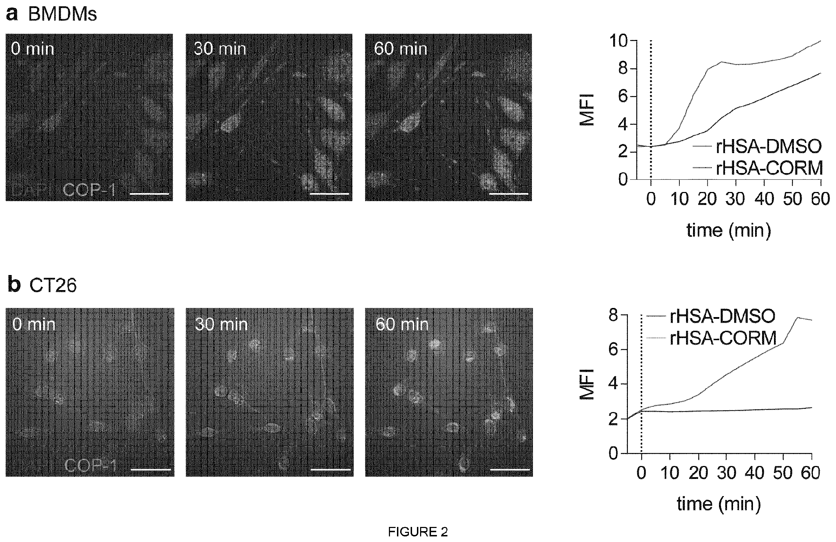

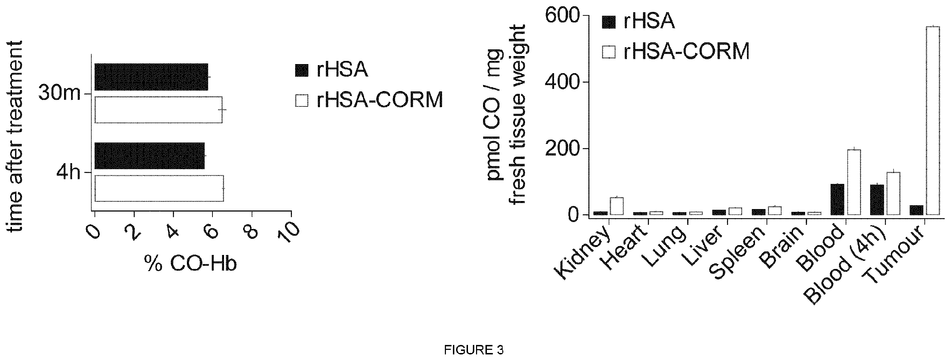

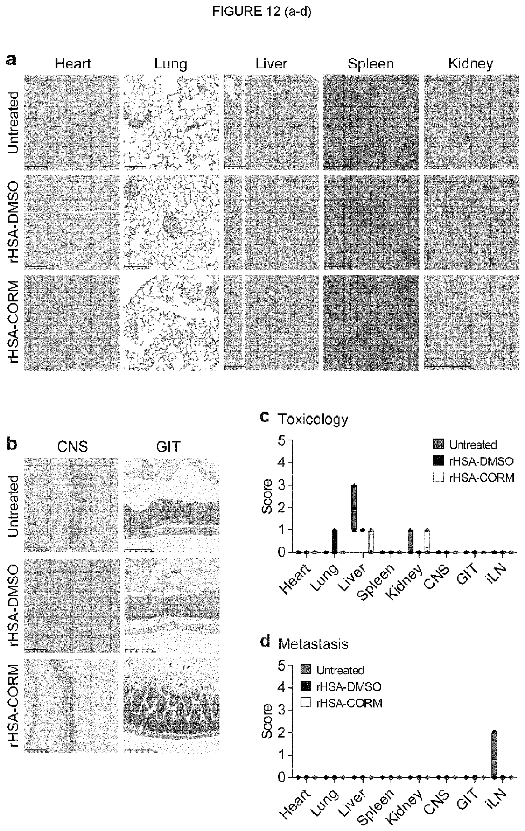

To determine if CO was effectively released from conjugates in CT26 cells, we screened CO levels through live imaging using a CO-sensitive dye named COP-1. [67] As expected, CO was able to accumulate in tumour cells over time (Fig. 2b). Furthermore, as CO has a higher affinity for haemoglobin than oxygen and may prevent tissue oxygenation, we estimated the blood content of carboxyhaemoglobin (CO-Hb) to assess potential CO poisoning in mice. We did not observe any increase in the formation of CO-Hb species upon intravenous administration of rHSA-CORMs nor have we detected any given signs of toxicity in tissues (Fig. 3, left; Fig. 12a-c). CO biodistribution is shown in Figure 3. Additionally, we observed some swelling in the inguinal lymph nodes (iLN) neighbouring tumours upon treatments that could imply an increase of immune cell recruitment in the site (Fig. 4c). Histology scores in different tissues also excluded the possibility of metastasis being at the root of iLN tumefaction, as no tumour cells were detected in the organs of rHSA-CORM-treated mice (Fig. 12d). CO release in BMDMs is also shown in Figure 2a.

### Tumour size and survival

It was demonstrated that CO treatment was effective in attenuating tumour growth and in prolonging overall survival of mice. Figure 4a shows a schematic of the experiment. Figure 4b clearly demonstrates that survival is significantly improved as a result of treatment with rHSA-CORM when compared to rHSA-DMSO treatment (p=0.0034). Figure 4b also shows that CT26 tumour growth is slowed by rHSA-CORM when compared to rHSA-DMSO treatment, with this being further demonstrated in Figure 4c in which rHSA-CORM treated tumours are considerably smaller than rHSA-DMSO treated tumours at day 13. Tumour weight is shown graphically in Figure 4d. It is also shown in Figure 4c that inguinal lymph nodes (iLN) at day 13 were larger after treatment with rHSA-CORM when compared to rHSA-DMSO treatment. This may suggest that rHSA-CORM treated mice had initiated a stronger immune response to the tumour cells.

rHSA-CORMs were equally effective in another mouse model of colon carcinoma using MC38 cells in C57BL/c mice.

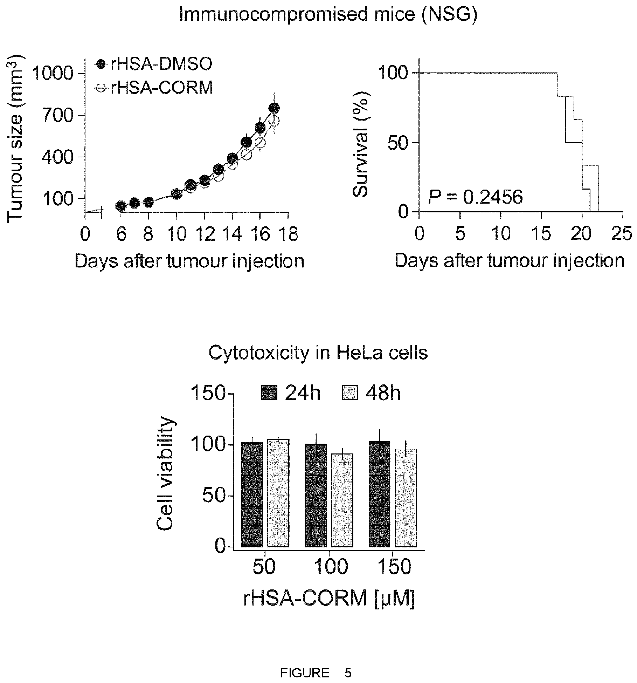

To assess whether CO-mediated effects actually relied on the immune system, we induced the same tumours in immunocompromised mice (NSG). As we could neither observe any differences between treatment groups nor direct cytotoxicity in CT26 cells (Fig. 5), we concluded that CO-mediated anti-tumour effects are immune-dependent. As shown in Fig. 5, there is no significant difference in tumour size, survival or cell viability between rHSA-CORM and rHSA-DMSO treatment in immunocompromised NSG mice. This suggests that CORM-conjugate treatment is most applicable to immunosuppressive cancer.

### Analysis of inflammatory response and immune response

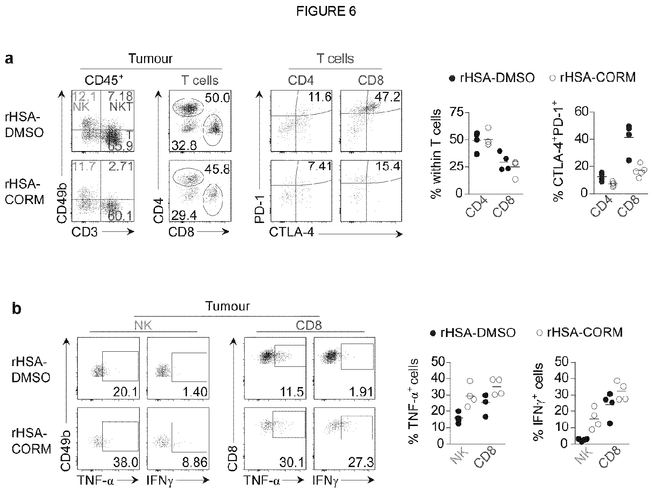

It is demonstrated in Figure 6a,b that CO-treated mice exhibited a reduction in the expression of immune-checkpoint markers CTLA-4 and PD-1 in tumour-infiltrating T cells (Fig. 6a), and also shows that CO treatments increase the abundance of cells expressing TNF-α and IFNγ (Fig. 6b). This indicates that CO treatment is likely to be effective in immunosuppressive cancers which are characterised by a reduced T-cell response, as both CTLA-4 and PD-1 inhibit T-cell action.

Figure 7a shows that CO affects the production of different cytokines and chemokines in tumours. Notably, CO treatment showed a dramatic reduction in the level of IL-20. (A) Protein quantification of cytokines (left) and chemokines (right) in tumours after rHSA-CORM treatments (rHSA-CORM, n=3; rHSA-DMSO, n=3).

As shown in Figs. 6a,b, we observed that despite not having differences in tumour-infiltrating lymphocyte abundance, the expression of CTLA-4 and PD-1, markers of suppression and exhaustion, was reduced in T cells whereas an increase in tumour-suppressing TNF-α- and IFNγ-producing CD8 and NK cells was observed after treatments. Such result reveals how CO is able to reduce the expression checkpoint proteins that prevent immune responses from being too strong. In order to determine on which kind of T cell subset these effects relied on, we eliminated CD4 and CD8 T cells in vivo using depleting antibodies. We observed that although CD4 T cells were redundant for CO-treatments, depletion of CD8 T cells reversed the effects of rHSA-CORM, suggesting that CO-mediated effects are CD8-dependent (Fig. 7b). Furthermore, as CO slightly affected the expression of ICM we hypothesised that ICB could synergise with rHSA-CORM treatments, further potentiating anti-tumour responses. Indeed, more tumours regressed in mice receiving combination treatment (COMBO) than ICB alone, hence improving survival counts (Fig. 7c). After mice cleared tumours completely, we rechallenged them with the same tumour cells in the opposite flanks. Strikingly, none of the mice previously treated with ICB and rHSA-CORM developed tumours (Fig. 7d).

### Macrophage response to CO

We evaluated the CO-mediated effects in the myeloid compartment, in particular tumour-associated macrophages and monocytes. MHC-II+ cells such as macrophages have the role of presenting antigens to T cells. However, in the context of tumours, TAMs skew to suppress T cells rather than activating them, which makes these macrophages detrimental in cancer treatment [2]. TAMs suppress T cell antitumour immunity and promote tumour development.

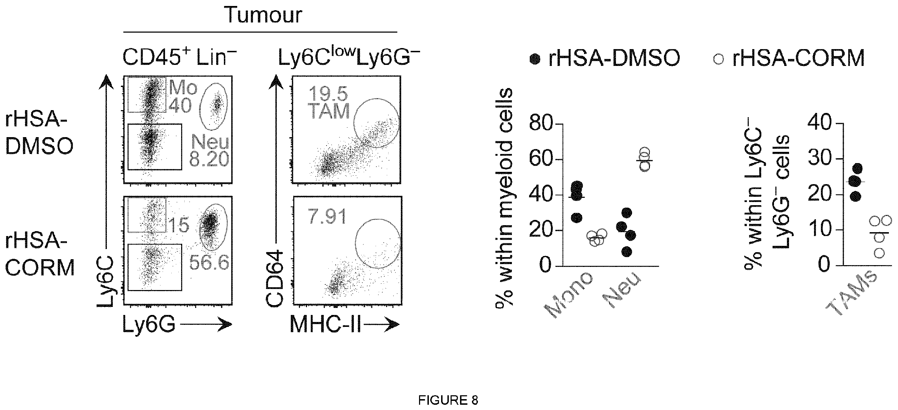

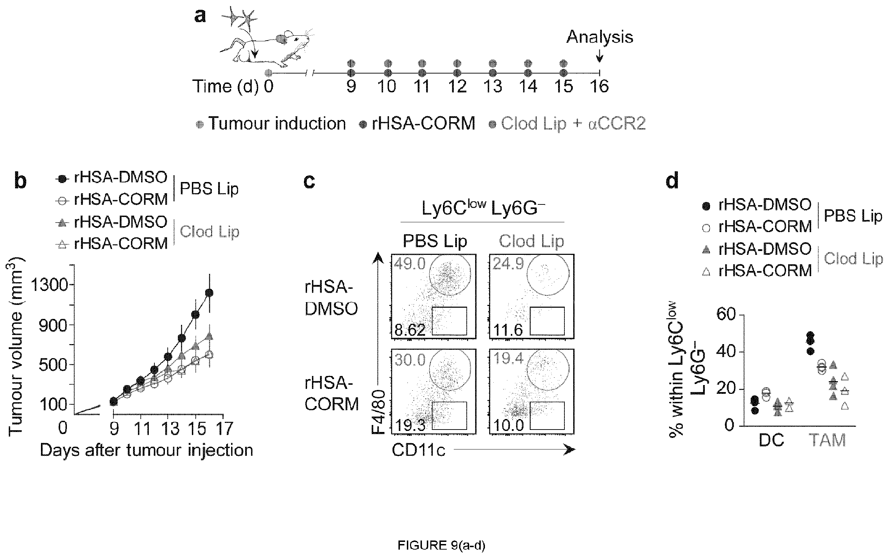

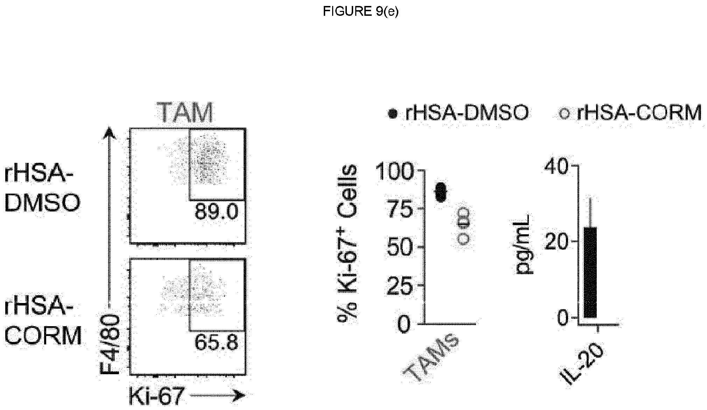

It is shown in Figure 8 that CO treatments reduce the abundance of monocytes and TAMs in tumours (rHSA-CORM, n=4; rHSA-DMSO, n=4). Interestingly, we also observed that CO treatments increased the abundance of neutrophils, possibly denoting an improvement in immune performance (Fig 8). Furthermore, Figure 9a-d shows that depletion of macrophages mimics the effects of CO treatments in CT26 tumours. (rHSA-CORM, n=4; rHSA-DMSO, n=4; rHSA-CORM+Clod Lip, n=4; rHSA-DMSO+Clod Lip, n=4). These macrophages were less proliferative, as denoted by a reduced Ki-67 expression (Fig. 9e), and depletion using clodronate liposomes mimicked the likes of CO in hampering tumour growth (Fig. 9b). This provides further evidence to show that that CO treatment is beneficial for the treatment of immunosuppressive cancers which are characterised by a reduced T-cell response.

We also saw a complete abrogation of IL-20 production in tumours after CO treatments (Fig. 7a and 9e). IL-20 is a pro-inflammatory cytokine from the IL-10 family associated with cancer progression, with monocytes and macrophages being its main producers. Therefore, CO-mediated anticancer effects may rely on macrophage depletion.

Following this idea, we sought to determine if CO was involved in macrophage homeostasis. To determine if the source of reduction in absolute cell numbers were due effects in macrophage proliferation or apoptosis, cells were marked for Ki-67 and Annexin V, respectively. No differences were observed in macrophage proliferation, contrarily to what was previously observed in vivo (Fig. 10e). Additionally, a reduction in apoptosis was observed with rHSA-CORM, as seen by the concomitant staining of Annexin V and Viability dye (Fig. 10f) which is inconsistent with the CO-mediated reduction of the number of cells. Therefore, CO is likely to affect viability of progenitors before differentiation and not macrophages at this concentration.

We differentiated macrophages from bone marrow (bone marrow-derived macrophages, "BMDM") in vitro and incubated them with rHSA-CORM or rHSA-DMSO (Fig. 10a). When monocytes were allowed to settle and start differentiating before adding rHSA-CORM, no differences occurred in terms of monocyte-to-macrophage differentiation, despite differences in total cell numbers (Fig. 10b-d). Therefore, we reasoned that CO might affect the viability of progenitors before differentiation and not in macrophages at such a low concentration (1µM).

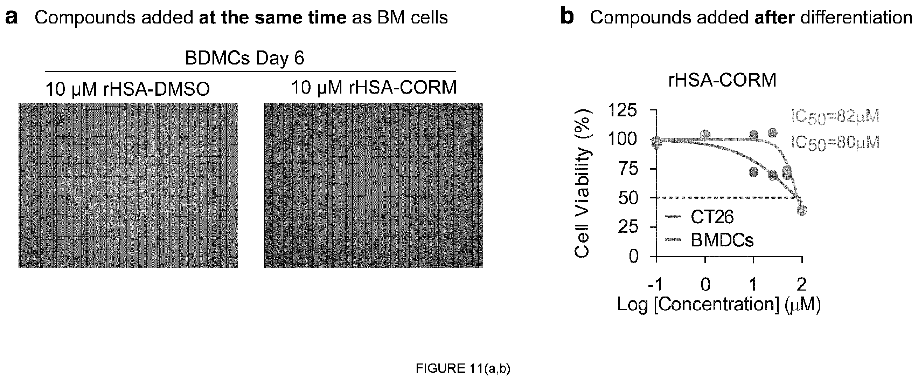

Cytotoxicity assays of differentiated BMDMs demonstrate that macrophages have a higher threshold of resistance than monocytes, displaying an GI50 of 80 µM. In contrast, using concentrations as low as 5 or 10 µM led to near-complete cell death in cultures, if compounds were added at the same time as BM cells (Fig. 11a). Hence, it appears that CO does not impair macrophage differentiation but rather weakens the viability of BM progenitors, leading to reduced numbers of macrophages. Further, CO affected macrophage polarization by reducing the expression of M1, M2a and M2c lineage markers (Fig. 10h). However, these effects may be secondary to polarisation and due to a reduced viability in macrophages.

Collectively, these results indicate that CO is effective in attenuating tumour growth and in prolonging overall survival of mice in an immune-dependent fashion, showing its promise as a treatment of immunosuppressive cancer. Currently available immunotherapeutic strategies are not always effective against cancer and relapses may occur even when working at the outset of treatments. CO presents itself as a new and versatile anticancer drug that may not only recapitulate the effects of immune-checkpoint inhibitors, but may also deplete cells that foster the immune-repressive environment of tumours. Consequently, CO may stand as a superior therapeutic alternative to conventional treatments.

### References

[1]. Bray, F. et al. CA. Cancer J. Clin. 68: 394-424 (2018).
[2]. Gajewski, T. F. et al. Nat. Immunol. 14, 1014-22 (2013).
[3]. Farkona, S. Et al. BMC Med. 14, 73 (2016).
[4]. Khalil, D. N. et al. Nat. Rev. Clin. Oncol. 13, 273-290 (2016).
[5]. Kersten, K. et al. Frontiers in Immunology 6, (2015).
[6]. Pitt, J. M. et al. Immunity 44, 1255-1269 (2016).
[7]. Amano, M. T. & Camara, N. O. Med. Gas. Res. 3, 1 (2013).
[8]. Motterlini, R. & Otterbein, L. E. Nat. ver. Drug Discov. 9, 728-743 (2010).
[9]. Chaves-Ferreira, M. et al. Angew. Chem. Int. Ed. Engl. 1172-1175 (2014).
[10]. Szabo, C. NaverRev. Drug Discov. 15, 185-203 (2015).
[11]. Kawahara, B. et al. Chem. Sci. ,11, 467-473 (2020)
[12]. Hoogenboezem, E. N. & Duvall C. L. Adv. Drug Deliv. Rev. 130 73-89 (2018).
[13] A Xia, et al. Front Immunol. 2019; 10: 1719.
[14] Marin-Acevedo J. A. et al. J Hematol Oncol. 2018; 11: 39.
[15] Nowicki T. S. et al. Cancer J. 2018 Jan-Feb; 24(1): 47-53.
[16] Rowshanravan B. et al. Blood. 2018 Jan 4; 131(1): 58-67.
[17] Ardolino L. and Joshua A. Aust Prescr. 2019 Apr; 42(2): 62-67.
[18] Ismailova A et al. Bioinorg Chem Appl. 2018; 2018: 8547364.
[19] Vreman HJ et al. Anal. Biochem. 2005; 341:280-289.
[20] Hodi FS, O'Day SJ, McDermott DF, et al. N Engl J Med. 2010;363:711-723.
[21] WeberJS, D'Angelo SP, Minor D, et al. Lancet Oncol. 2015;16:375-384.
[22] Robert C, Long GV, Brady B, et al. N Engl J Med. 2015;372:320-330.
[23] Ribas A, Puzanov I, Dummer R, et al. Lancet Oncol. 2015;16:908-918.
[24] Robert C, Schachter J, Long GV, et al. N Engl J Med. 2015;372:2521-2532.
[25] Garon EB, Rizvi NA, Hui R, et al. N Engl J Med. 2015;372:2018-2028.
[26] Herbst RS, Baas P, Kim DW, et al. Lancet. 2016;387:1540-1550.
[27] Reck M, Rodriguez-Abreu D, Robinson AG, et al. N Engl J Med. 2016;375:1823-1833.
[28] Chen R, Zinzani PL, Fanale MA, et al. J Clin Oncol. 2017;35:2125-2132.
[29] Chow LQM, Haddad R, Gupta S, et al. J Clin Oncol. 2016;34:3838-3845.
[30] Bellmunt J, de Wit R, Vaughn DJ, et al. N Engl J Med. 2017;376:1015-1026.
[31] Zhu AX, Finn RS, Edeline J, et al. Lancet Oncol. 2018;19:940-952.
[32] Zinzani PL, Thieblemont C, Melnichenko V, et al. Blood. 2017;130(Suppl 1):2833.
[33] Borghaei H, Paz-Ares L, Horn L, et al. N Engl J Med. 2015;373:1627-1639.
[34] Chung HC, Schellens JHM, Delord J-P, et al. J Clin Oncol. 2018;36(15_suppl):5522.
[35] Fuchs CS, Doi T, Jang RW, et al. JAMA Oncol. 2018;4:e180013.
[36] Prasad V, Kaestner V, Mailankody S. JAMA Oncol. 2018;4:157-158.
[37] Nghiem PT, Bhatia S, Lipson EJ, et al. N Engl J Med. 2016;374:2542-2552.
[38] Brahmer J, Reckamp KL, Baas P, et al. N Engl J Med. 2015;373:123-135.
[39] Motzer RJ, Escudier B, McDermott DF, et al. N Engl J Med. 2015;373:1803-1813.
[40] Sharma P, Retz M, Siefker-Radtke A, et al. Lancet Oncol. 2017;18:312-322.
[41] El-Khoueiry AB, Sangro B, Yau T, et al. 2017;389:2492-2502.
[42] Overman MJ, McDermott R, Leach JL, et al. Lancet Oncol. 2017;18:1182-1191.
[43] Younes A, Santoro A, Shipp M, et al. Lancet Oncol. 2016;17:1283-1294.
[44] Ready N, Farago AF, de Braud F, et al. J Thorac Oncol. 2019;14:237-244.
[45] Migden MR, Rischin D, Schmults CD, et al. N Engl J Med. 2018;379:341-351.
[46] Powles T, Durán I, van der Heijden MS, et al. Lancet. 2018; 391:748-757.
[47] Rittmeyer A, Barlesi F, Waterkamp D, et al. Lancet. 2017;389:255-265.
[48] Kaufman HL, Russell J, Hamid O, et al. Lancet Oncol. 2016;17:1374-1385.
[49] Patel MR, Ellerton J, Infante JR, et al. Lancet Oncol. 2018;19:51-64.
[50] Antonia SJ, Villegas A, Daniel D, et al. N Engl J Med. 2017;377:1919-1929.
[51] Powles T, O'Donnell PH, Massard C, et al. JAMA Oncol. 2017;3:e172411.
[52] Schumacher TN, Schreiber RD. Science. 2015;348:69-74.
[53] Rizvi NA, Hellmann MD, Snyder A, et al. Science. 2015;348:124-128.
[54] Rizvi H, Sanchez-Vega F, La K, et al. J Clin Oncol. 2018;36:633-641.
[55] Yarchoan M, Hopkins A, Jaffee EM. N Engl J Med. 2017;377:2500-2501.
[56] Tumeh PC, Harview CL, Yearley JH, et al. Nature. 2014;515:568-571.
[57] Van Allen EM, Miao D, Schilling B, et al. Science. 2015;350:207-211.
[58] Fares CM, et al. American Society of Clinical Oncology Educational Book 39.147-164.
[59] Topalian SL, Hodi FS, Brahmer JR, et al. N Engl J Med. 2012;366:2443-2454.
[60] Martin AM, Nirschl TR, Nirschl CJ, et al. Prostate Cancer Prostatic Dis. 2015;18:325-332.
[61] Kallergi, G et al. Ther Adv Med Oncol. 2018, 10: 1-11.
[62] Gomez-Roca CA et al. Ann. Oncol. 2019. 30(8):1381-1392.
[63] Bercovici N et al. Front Immunol. 2019;10:1563.
[64] Wunder A. et al. Int J Oncol. 1997 Sep;11(3):497-507
[65] Loboda A, et al. Vasc. Pharmacol. 2015; 74:11-22.
[66] Yan H, et al. Small (2019) 15, 1904382.
[67] Michel BW, Lippert AR, Chang CJ. J Am Chem Soc. 2012 Sep 26;134(38):15668-71.

## Claims

1. A conjugate for use in a method of reducing tumour immunosuppression in a tumour tissue, said conjugate comprising one or more carbon monoxide releasing groups and a tumour-selective ligand, wherein the method comprises administering said conjugate and optionally administering a cancer immunotherapy, wherein the tumour tissue comprises the tumour and its microenvironment.

2. The conjugate for the use of claim 1, wherein the method comprises administering a cancer immunotherapy.

3. The conjugate for the use according to claim 1, wherein the tumour tissue is an immunosuppressive cancer, optionally wherein the immunosuppressive cancer is **characterised by** immune checkpoint protein mediated inhibition of T cell function.

4. The conjugate for the use according to claim 3, wherein the immune checkpoint protein is CTLA-4 or PD-1.

5. The conjugate for the use according to claim 3, wherein the immunosuppressive cancer is **characterised by** the presence of T-cell inhibitory cells in tumour tissue of the individual, optionally wherein the T-cell inhibitory cells are tumour-associated macrophages (TAMs).

6. The conjugate for the use according to any one of claims 1 to 5, wherein the carbon monoxide releasing groups are metal carbonyl groups, optionally wherein the metal carbonyl groups are mono-, di-, tri-, tetra- or penta-carbonyl metal groups, optionally wherein the carbon monoxide releasing group comprises a metal atom selected from ruthenium, molybdenum, cobalt, rhenium, and iron.

7. The conjugate for the use according to claim 6, wherein the conjugate has the formula Ligand-[Metal(CO)n1]n₂ where n₁ is between 1 and 5 and n₂ is between 1 and 30, optionally wherein the carbon monoxide releasing groups are ruthenium carbonyl groups, optionally wherein the conjugate has the formula Ligand-[Ru (CO)₂]n, where n is between 1 and 16, optionally wherein the carbon monoxide releasing groups are Ru^{II}(CO)₂.

8. The conjugate for the use according to any one of claims 1 to 7, wherein the conjugate comprises two or more carbon monoxide releasing groups, optionally wherein the conjugate is a nanoparticle encapsulating the carbon-monoxide-releasing groups, with the tumour selective ligand conjugated to the surface of the nanoparticle, optionally wherein the nanoparticle comprises a polymeric or inorganic core or shell.

9. The conjugate for the use of any one of claims 1 to 8, wherein the tumour selective ligand is a biopolymer, synthetic polymer, peptide, aptamer, or small molecule, optionally wherein the tumour selective ligand is a protein, optionally wherein the tumour selective ligand is a plasma protein, optionally wherein the tumour-selective ligand is an antibody, albumin, globulin, lipoprotein, or transferrin.

10. The conjugate for the use of claim 9, wherein the tumour-selective ligand is serum albumin or fragment thereof, which is human serum albumin; or wherein the tumour-selective ligand comprises an antibody or antigen-binding portion thereof, optionally wherein the antibody or antigen-binding portion thereof specifically binds a tumour antigen or specifically binds to PD-1 or CTLA4.

11. The conjugate for the use according to claim 9 or 10, wherein the tumour selective ligand is a protein, and the carbon monoxide releasing groups are attached to histidine residues in the protein by dative covalent bonds; or the tumour selective ligand is a protein and the conjugate is produced by reacting the protein with a CO releasing molecule comprising one or more metal carbonyl groups, such that one or more histidine residues of the protein are metalated with the metal carbonyl groups, optionally wherein the conjugate is produced by reacting the protein with CORM-3, such that one or more histidine residues of the protein are metalated with ruthenium dicarbonyl groups.

12. The conjugate for the use according to any one of claims 3 to 11, wherein the immunosuppressive cancer is a solid cancer such as sarcoma, carcinoma, or lymphoma; optionally wherein the cancer is skin cancer, melanoma, bladder cancer, brain cancer, breast cancer, uterus cancer, oral cancer, ovary cancer, prostate cancer, lung cancer, colorectal cancer, cervical cancer, liver cancer, head and neck cancer, oesophageal cancer, pancreas cancer, renal cancer, adrenal cancer, stomach cancer, testicular cancer, cancer of the gall bladder and biliary tracts, thyroid cancer, thymus cancer, and bone cancer; optionally wherein the cancer is a primary tumour or the cancer is metastatic.

13. The conjugate for the use according to any one of claims 1 to 12, wherein the conjugate is to be administered once or twice per week or once or twice per month, and/or wherein the conjugate is to be administered for at least four weeks, and/or wherein the CO releasing conjugate is to be administered intravenously.

14. The conjugate for the use according to any one of claims 1 to 13, wherein conjugate is to be administered to the individual in combination with cancer immunotherapy; optionally wherein the cancer immunotherapy is selected from immune checkpoint inhibitor therapy, cytokine therapy, immune co-stimulatory therapy, and adoptive cell therapy; optionally wherein:
(a) the cancer immunotherapy is an immune checkpoint inhibitor selected from an anti-CTLA-4 antibody, an anti-PD-1 antibody, and an anti-PD-L1 antibody, optionally wherein the immune checkpoint inhibitor is ipilimumab, tremelimumab, nivolumab, pembrolizumab, durvalumab, atezolizumab, and avelumab; or
(b) wherein the cancer immunotherapy is an immune co-stimulatory therapy, which is optionally a CD28 agonist or an OX40 agonist; or
(c) wherein the cancer immunotherapy is an adoptive cell therapy, optionally a chimeric antigen receptor (CAR) T cell therapy.

15. The conjugate for the use according to any one of claims 1 to 14, wherein the therapeutic regimen includes one or more of (a) administration of the conjugate for one to four weeks before initiating an immunotherapy regimen; (b) administration of the conjugate for one or more weeks or months after the completion of a cancer immunotherapy regimen; and (c) concomitant therapy.

16. The conjugate for the use according to any one of claims 1 to 15, wherein the patient was previously unresponsive to, only partially responsive to, or had become resistant to, an immunotherapy, which is optionally a checkpoint inhibitor therapy; optionally wherein the patient is refractory to CTLA-4 or PD-1 blockade therapy.

## Patentansprüche

1. Ein Konjugat zur Verwendung in einem Verfahren zur Verringerung von Tumor-Immunsuppression in einem Tumorgewebe, jenes Konjugat umfassend eine oder mehr Kohlenstoffmonoxid-freisetzende Gruppen und einen tumorselektiven Liganden, wobei das Verfahren Verabreichen jenes Konjugats und optional Verabreichen einer Krebsimmuntherapie umfasst, wobei das Tumorgewebe den Tumor und seine Mikroumgebung umfasst.

2. Das Konjugat zur Verwendung aus Anspruch 1, wobei das Verfahren Verabreichen einer Krebsimmuntherapie umfasst.

3. Das Konjugat zur Verwendung gemäß Anspruch 1, wobei das Tumorgewebe ein immunsuppressiver Krebs ist, optional wobei der immunsuppressive Krebs **gekennzeichnet ist durch** Immuncheckpointprotein-vermittelte Hemmung der T-Zell-Funktion.

4. Das Konjugat zur Verwendung gemäß Anspruch 3, wobei das Immuncheckpointprotein CTLA-4 oder PD-1 ist.

5. Das Konjugat zur Verwendung gemäß Anspruch 3, wobei der immunsuppressive Krebs **gekennzeichnet ist durch** die Gegenwart von T-Zell-inhibitorischen Zellen in Tumorgewebe des Individuums, optional wobei die T-Zell-inhibitorischen Zellen Tumor-assoziierte Makrophagen (TAMs) sind.

6. Das Konjugat zur Verwendung gemäß irgendeinem der Ansprüche 1 bis 5, wobei die Kohlenstoffmonoxid-freisetzenden Gruppen Metallcarbonylgruppen sind, optional wobei die Metallcarbonylgruppen Mono-, Di-, Tri-, Tetra- oder Pentacarbonyl-Metallgruppen sind, optional wobei die Kohlenstoffmonoxid-freisetzende Gruppe ein Metallatom ausgewählt aus Ruthenium, Molybdän, Kobalt, Rhenium und Eisen umfasst.

7. Das Konjugat zur Verwendung gemäß Anspruch 6, wobei das Konjugat die Formel Ligand-[Metall(CO)n1]n₂ hat, wobei n₁ zwischen 1 und 5 ist und n₂ zwischen 1 und 30 ist, optional wobei die Kohlenstoffmonoxid-freisetzenden Gruppen Rutheniumcarbonyl-Gruppen sind, optional wobei das Konjugat die Formel Ligand-[Ru (CO)₂]n hat, wobei n zwischen 1 und 16 ist, optional wobei die Kohlenstoffmonoxid-freisetzenden Gruppen Ru^{II}(CO)₂ sind.

8. Das Konjugat zur Verwendung gemäß irgendeinem der Ansprüche 1 bis 7, wobei das Konjugat zwei oder mehr Kohlenstoffmonoxid-freisetzende Gruppen umfasst, optional wobei das Konjugat ein Nanopartikel ist, das die Kohlenstoffmonoxid-freisetzenden Gruppen umschließt, wobei der tumorselektive Ligand an die Oberfläche des Nanopartikels konjugiert ist, optional wobei das Nanopartikel einen polymeren oder anorganischen Kern oder Schale umfasst.

9. Das Konjugat zur Verwendung gemäß irgendeinem der Ansprüche 1 bis 8, wobei der tumorselektive Ligand ein Biopolymer, synthetisches Polymer, Peptid, Aptamer oder kleines Molekül ist, optional wobei der tumorselektive Ligand ein Protein ist, optional wobei der tumorselektive Ligand ein Plasmaprotein ist, wobei der tumorselektive Ligand ein Antikörper, Albumin, Globulin, Lipoprotein oder Transferrin ist.

10. Das Konjugat zur Verwendung aus Anspruch 9, wobei der tumorselektive Ligand Serumalbumin oder Fragment davon ist, welches humanes Serumalbumin ist; oder wobei der tumorselektive Ligand einen Antikörper oder antigenbindenden Teil davon umfasst, optional wobei der Antikörper oder antigenbindende Teil davon spezifisch ein Tumorantigen bindet oder spezifisch PD-1 oder CTLA4 bindet.

11. Das Konjugat zur Verwendung gemäß Anspruch 9 oder 10, wobei der tumorselektive Ligand ein Protein ist und die Kohlenstoffmonoxid-freisetzenden Gruppen über gebende kovalente Bindungen an Histidinresten im Protein angebracht sind; oder der tumorselektive Ligand ein Protein ist und das Konjugat durch Reaktion des Proteins mit einem CO-freisetzenden Molekül umfassend eine oder mehr Metallcarbonyl-Gruppen erzeugt wird, so dass ein oder mehr Histidinreste des Proteins mit Metallcarbonyl-Gruppen metalliert werden, optional wobei das Konjugat durch Reaktion des Proteins mit CORM-3 erzeugt wird, so dass ein oder mehr Histidinreste des Proteins mit Rutheniumdicarbonyl-Gruppen metalliert werden.

12. Das Konjugat zur Verwendung gemäß irgendeinem der Ansprüche 3 bis 11, wobei der immunsuppressive Krebs ein solider Krebs wie etwa Sarkom, Karzinom oder Lymphom ist; optional wobei der Krebs Hautkrebs, Melanom, Blasenkrebs, Hirnkrebs, Brustkrebs, Gebärmutterkrebs, Mundkrebs, Eierstockkrebs, Prostatakrebs, Lungenkrebs, Darmkrebs, Gebärmutterhalskrebs, Leberkrebs, Kopf- und Halskrebs, Speiseröhrenkrebs, Bauchspeicheldrüsenkrebs, Nierenkrebs, Nebennierenkrebs, Magenkrebs, Hodenkrebs, Krebs der Gallenblase oder Gallengänge, Schilddrüsenkrebs, Thymuskrebs und Knochenkrebs ist; optional wobei der Krebs ein primärer Tumor ist oder der Krebs metastatisch ist.

13. Das Konjugat zur Verwendung gemäß irgendeinem der Ansprüche 1 bis 12, wobei das Konjugat ein- oder zweimal pro Woche oder ein- oder zweimal pro Monat zu verabreichen ist, und/oder wobei das Konjugat mindestens vier Wochen lang zu verabreichen ist, und/oder wobei das CO-freisetzende Konjugat intravenös zu verabreichen ist.

14. Das Konjugat zur Verwendung gemäß irgendeinem der Ansprüche 1 bis 13, wobei das Konjugat dem Individuum in Kombination mit einer Krebsimmuntherapie zu verabreichen ist; optional wobei die Krebsimmuntherapie ausgewählt ist aus Immuncheckpointinhibitor-Therapie, Zytokintherapie, kostimulatorischer Immuntherapie und adoptiver Zelltherapie; optional wobei:
(a) die Krebsimmuntherapie ein Immuncheckpointinhibitor ausgewählt ist aus einem anti-CTLA-4-Antikörper, einem anti-PD-1-Antikörper und einem anti-PD-L1-Antikörper, optional wobei der Immuncheckpointinhibitor Ipilimumab, Tremelimumab, Nivolumab, Pembrolizumab, Durvalumab, Atezolizumab und Avelumab ist; oder
(b) wobei die Krebsimmuntherapie eine kostimulatorische Immuntherapie ist, die optional ein CD28-Agonist oder ein OX40-Agonist ist; oder
(c) wobei die Krebsimmuntherapie adoptive Zelltherapie ist, optional eine Therapie mit T-Zellen mit chimärem Antigenrezeptor (CAR).

15. Das Konjugat zur Verwendung gemäß irgendeinem der Ansprüche 1 bis 14, wobei das therapeutische Regime eines oder mehr umfasst von (a) Verabreichung des Konjugats über eine bis vier Wochen vor Einsetzen eines Immuntherapieregimes; (b) Verabreichung des Konjugats über eine bis vier Wochen oder Monate nach Abschluss eines Krebsimmuntherapieregimes; und (c) begleitende Therapie.

16. Das Konjugat zur Verwendung gemäß irgendeinem der Ansprüche 1 bis 14, wobei der Patient vorher auf eine Immuntherapie nicht ansprach, nur teilweise ansprach oder dafür resistent geworden war, welche optional eine Checkpointinhibitor-Therapie ist; optional wobei der Patient gegenüber CTLA-4- oder PD-1-Blockadetherapie resistent ist.

## Revendications

1. Conjugué pour une utilisation dans un procédé de réduction de l'immunosuppression tumorale dans un tissu tumoral, ledit conjugué comprenant un ou plusieurs groupes libérant du monoxyde de carbone et un ligand sélectif de tumeur, dans lequel le procédé comprend l'administration dudit conjugué et facultativement l'administration d'une immunothérapie anticancéreuse, dans lequel le tissu tumoral comprend la tumeur et son microenvironnement.

2. Conjugué pour l'utilisation selon la revendication 1, dans lequel le procédé comprend l'administration d'une immunothérapie anticancéreuse.

3. Conjugué pour l'utilisation selon la revendication 1, dans lequel le tissu tumoral est un cancer immunosuppresseur, facultativement dans lequel le cancer immunosuppresseur est **caractérisé par** une inhibition de la fonction des lymphocytes T médiée par une protéine de point de contrôle immunitaire.

4. Conjugué pour l'utilisation selon la revendication 3, dans lequel la protéine de point de contrôle immunitaire est CTLA-4 ou PD-1.

5. Conjugué pour l'utilisation selon la revendication 3, dans lequel le cancer immunosuppresseur est **caractérisé par** la présence de cellules inhibitrices des lymphocytes T dans le tissu tumoral de l'individu, facultativement dans lequel les cellules inhibitrices des lymphocytes T sont des macrophages associés à la tumeur (TAM).

6. Conjugué pour l'utilisation selon l'une quelconque des revendications 1 à 5, dans lequel les groupes libérant du monoxyde de carbone sont des groupes métal carbonyle, facultativement dans lequel les groupes métal carbonyle sont des groupes métal mono-, di-, tri-, tétra- ou penta-carbonyle, facultativement dans lequel le groupe libérant du monoxyde de carbone comprend un atome de métal choisi parmi le ruthénium, le molybdène, le cobalt, le rhénium et le fer.

7. Conjugué pour l'utilisation selon la revendication 6, dans lequel le conjugué a la formule Ligand-[Métal(CO)n1]n₂ où n₁ est compris entre 1 et 5 et n₂ est compris entre 1 et 30, facultativement dans lequel les groupes libérant du monoxyde de carbone sont des groupes ruthénium carbonyle, facultativement dans lequel le conjugué a la formule Ligand-[Ru (CO)₂]n, où n est compris entre 1 et 16, facultativement dans lequel les groupes libérant du monoxyde de carbone sont Ru^{II}(CO)₂.

8. Conjugué pour l'utilisation selon l'une quelconque des revendications 1 à 7, dans lequel le conjugué comprend deux groupes libérant du monoxyde de carbone ou plus, facultativement dans lequel le conjugué est une nanoparticule encapsulant les groupes libérant du monoxyde de carbone, avec le ligand sélectif de tumeur conjugué à la surface de la nanoparticule, facultativement dans lequel la nanoparticule comprend un noyau ou une enveloppe polymère ou inorganique.

9. Conjugué pour l'utilisation selon l'une quelconque des revendications 1 à 8, dans lequel le ligand sélectif de tumeur est un biopolymère, un polymère synthétique, un peptide, un aptamère ou une petite molécule, facultativement dans lequel le ligand sélectif de tumeur est une protéine, facultativement dans lequel le ligand sélectif de tumeur est une protéine plasmatique, facultativement dans lequel le ligand sélectif de tumeur est un anticorps, une albumine, une globuline, une lipoprotéine ou une transferrine.

10. Conjugué pour l'utilisation selon la revendication 9, dans lequel le ligand sélectif de tumeur est de l'albumine sérique ou un fragment de celle-ci, qui est de l'albumine sérique humaine ; ou dans lequel le ligand sélectif de tumeur comprend un anticorps ou une partie de liaison à l'antigène de celui-ci, facultativement dans lequel l'anticorps ou la partie de liaison à l'antigène de celui-ci se lie spécifiquement à un antigène tumoral ou se lie spécifiquement à PD-1 ou CTLA4.

11. Conjugué pour l'utilisation selon la revendication 9 ou 10, dans lequel le ligand sélectif de tumeur est une protéine, et les groupes libérant du monoxyde de carbone sont liés à des résidus d'histidine dans la protéine par des liaisons covalentes datives ; ou le ligand sélectif de tumeur est une protéine et le conjugué est produit par réaction de la protéine avec une molécule libérant du CO comprenant un ou plusieurs groupes métal carbonyle, de sorte qu'un ou plusieurs résidus d'histidine de la protéine soient métallés avec les groupes métal carbonyle, facultativement dans lequel le conjugué est produit par réaction de la protéine avec CORM-3, de sorte qu'un ou plusieurs résidus d'histidine de la protéine soient métallés avec des groupes ruthénium dicarbonyle.

12. Conjugué pour l'utilisation selon l'une quelconque des revendications 3 à 11, dans lequel le cancer immunosuppresseur est un cancer solide tel qu'un sarcome, un carcinome ou un lymphome ; facultativement dans lequel le cancer est un cancer de la peau, un mélanome, un cancer de la vessie, un cancer du cerveau, un cancer du sein, un cancer de l'utérus, un cancer buccal, un cancer de l'ovaire, un cancer de la prostate, un cancer du poumon, un cancer colorectal, un cancer du col de l'utérus, un cancer du foie, un cancer de la tête et du cou, un cancer de l'œsophage, un cancer du pancréas, un cancer du rein, un cancer des surrénales, un cancer de l'estomac, un cancer du testicule, un cancer de la vésicule biliaire et des voies biliaires, un cancer de la thyroïde, un cancer du thymus et un cancer osseux ; facultativement dans lequel le cancer est une tumeur primitive ou le cancer est métastatique.

13. Conjugué pour l'utilisation selon l'une quelconque des revendications 1 à 12, dans lequel le conjugué doit être administré une ou deux fois par semaine ou une ou deux fois par mois, et/ou dans lequel le conjugué doit être administré pendant au moins quatre semaines, et/ou dans lequel le conjugué libérant du CO doit être administré par voie intraveineuse.

14. Conjugué pour l'utilisation selon l'une quelconque des revendications 1 à 13, dans lequel le conjugué doit être administré à l'individu en association avec une immunothérapie anticancéreuse ; facultativement dans lequel l'immunothérapie anticancéreuse est choisie parmi une thérapie par inhibiteur de point de contrôle immunitaire, une thérapie par cytokines, une thérapie de co-stimulation immunitaire et une thérapie cellulaire adoptive ; facultativement dans lequel :
(a) l'immunothérapie anticancéreuse est un inhibiteur de point de contrôle immunitaire choisi parmi un anticorps anti-CTLA-4, un anticorps anti-PD-1 et un anticorps anti-PD-L1, facultativement dans lequel l'inhibiteur de point de contrôle immunitaire est l'ipilimumab, le trémélimumab, le nivolumab, le pembrolizumab, le durvalumab, l'atézolizumab et l'avélumab ; ou
(b) dans lequel l'immunothérapie anticancéreuse est une thérapie de co-stimulation immunitaire, qui est facultativement un agoniste de CD28 ou un agoniste d'OX40 ; ou
(c) dans lequel l'immunothérapie anticancéreuse est une thérapie cellulaire adoptive, facultativement une thérapie par lymphocytes T à récepteur antigénique chimérique (CAR).

15. Conjugué pour l'utilisation selon l'une quelconque des revendications 1 à 14, dans lequel le régime thérapeutique comporte une ou plusieurs parmi (a) l'administration du conjugué pendant une à quatre semaines avant l'instauration d'un régime d'immunothérapie ; (b) l'administration du conjugué pendant une ou plusieurs semaines ou mois après l'achèvement d'un régime d'immunothérapie anticancéreuse ; et (c) un traitement concomitant.

16. Conjugué pour l'utilisation selon l'une quelconque des revendications 1 à 15, dans lequel le patient n'a pas répondu auparavant, n'a répondu que partiellement ou est devenu résistant à une immunothérapie, qui est facultativement une thérapie par inhibiteur de point de contrôle ; facultativement dans lequel le patient est réfractaire à une thérapie par blocage de CTLA-4 ou PD-1.
